(19) Europäisches Patentamt
European Patent Office
Office européen des brevets

(11) **EP 4 567 415 A1**

(12) **EUROPEAN PATENT APPLICATION**
published in accordance with Art. 153(4) EPC

(43) Date of publication:
**11.06.2025 Bulletin 2025/24**

(21) Application number: **23850080.5**

(22) Date of filing: **01.08.2023**

(51) International Patent Classification (IPC):
**G01N 27/327** (2006.01)

(52) Cooperative Patent Classification (CPC):
**G01N 27/327**

(86) International application number:
**PCT/JP2023/028101**

(87) International publication number:
**WO 2024/029526 (08.02.2024 Gazette 2024/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **03.08.2022 JP 2022124198**

(71) Applicant: **PHC HOLDINGS CORPORATION
Tokyo 100-8403 (JP)**

(72) Inventors:
• **SHIRAI, Akihito**
  **Toon-shi, Ehime 791-0395 (JP)**
• **NISHIYAMA, Masato**
  **Toon-shi, Ehime 791-0395 (JP)**

(74) Representative: **Eisenführ Speiser
Patentanwälte Rechtsanwälte PartGmbB
Postfach 10 60 78
28060 Bremen (DE)**

(54) **ELECTRODE, SENSOR, AND METHOD FOR PRODUCING SENSOR**

(57)    An electrode is provided that has a surface portion that allows a reagent layer having the designed dimensions to be formed very accurately over a specific area. The electrode (10) comprises a surface portion (11); a first region (110) that is formed on the surface portion (11), includes a first outer peripheral edge portion (111), and has a first surface free energy; a second region (120) that is formed on the surface portion (11), surrounds the first region (110), includes a second inner peripheral edge portion (121) in contact with the first outer peripheral edge portion (111) of the first region (110), and a second outer peripheral edge portion (122) that is positioned more to the outside than the second inner peripheral edge portion (121), and has a second surface free energy that is greater than the first surface free energy; and a third region (130) that surrounds the second region (120), includes a third inner peripheral edge portion (131) in contact with the second outer peripheral edge portion (122) of the second region (120), and has a third surface free energy that is less than the second surface free energy.

FIG. 7

**Description**

TECHNICAL FIELD

The present invention relates, for example, to an electrode, a sensor, and a method for manufacturing a sensor.

BACKGROUND ART

Electrochemical sensors have been used in the past to measure a target substance in a test sample, such as a cell culture fluid or a blood sample. Such sensors include, for example, an insulating substrate and a working electrode that is disposed on the surface of the substrate. The working electrode typically includes an electrode and a reagent layer that is disposed on the electrode and contains a reagent that participates in an oxidation-reduction reaction (such as an oxidoreductase or an electron carrier).

Patent Literature 1 discloses a biosensor that detects a target substance contained in a liquid sample, said biosensor including an insulating substrate having a recess formed in a portion thinner than the surrounding area, a working electrode and a counter electrode, at least one of which is disposed in the recess, and a reaction reagent that is disposed in the recess and reacts with a specific substance in the liquid sample. In a working example in Patent Literature 1, it is indicated that a circular recess is formed on the surface of a polyethylene terephthalate substrate, and after the working electrode is formed in the recess, the reagent layer is formed.

CITATION LIST

PATENT LITERATURE

Patent Literature 1: JP-A 2010-32501

SUMMARY

TECHNICAL PROBLEM

However, the following problem is encountered with the above-mentioned conventional sensor having a working electrode including an electrode and a reagent layer disposed in a recess in an insulating substrate.

The sensitivity at which an analyte is detected by a sensor having a working electrode depends on the amount and density of the reagent contained in the reagent layer on the working electrode, the contact surface area between the working electrode and the reagent layer, and so forth. Therefore, improving the accuracy of detection of an analyte by a sensor having a working electrode requires a reagent layer with uniform dimensions (surface area, thickness, shape) to be formed on the electrode. In the biosensor described in Patent Literature 1, the dimensions of the reagent layer on the working electrode and the contact surface area between the working electrode and the reagent layer can fluctuate depending on the position and shape of the electrode in the recess of the insulating substrate, so it can be difficult to accurately form a reagent layer of the designed dimensions in the specified region on the surface portion of the electrode.

It is an object of the present invention to provide an electrode having a surface portion that allows a reagent layer of the designed dimensions to be accurately formed in the specified region, as well as a sensor comprising this electrode, and a method for manufacturing this sensor.

SOLUTION TO PROBLEM

The present invention relates to a method for manufacturing an electrode to be disposed on an insulating substrate, said electrode comprising:

a surface portion that is positioned on the opposite side from the substrate when the electrode is disposed on the substrate;
a first region that is formed on the surface portion, includes a first outer peripheral edge portion, and has a first surface free energy;
a second region that is formed on the surface portion, surrounds the first region, includes a second inner peripheral edge portion in contact with the first outer peripheral edge portion of the first region, and a second outer peripheral edge portion positioned more to the outside than the second inner peripheral edge portion, and has a second surface free energy that is greater than the first surface free energy; and
a third region that is formed on the surface portion, surrounds the second region, includes a third inner peripheral edge portion in contact with the second outer peripheral edge portion of the second region, and has a third surface free energy that is less than the second surface free energy.

The present invention also relates to an electrode to be disposed on an insulating substrate, said electrode comprising:

a surface portion that is positioned on the opposite side from the substrate when the electrode is disposed on the substrate;
a first region that is formed on the surface portion, includes a first outer peripheral edge portion, and has a first surface free energy;
a second region that is formed on the surface portion, surrounds the first region, includes a second inner peripheral edge portion in contact with the first outer peripheral edge portion of the first region, and a second outer peripheral edge portion positioned more to the outside than the second inner peripheral edge portion, and has a second surface free energy that is greater than the first surface free energy; and
an insulating layer, at least a portion of which is disposed on the surface portion, and that includes an opening formed in the first region and the second region, having an inner peripheral edge portion that defines the second outer peripheral edge portion of the second region, and passing through in the thickness direction.

The present invention also relates to a sensor, comprising:

the above-mentioned electrode; and
a reagent layer that is disposed in the first region and the second region of the electrode, and includes an outer peripheral edge portion positioned on the second outer peripheral edge portion of the second region, and a reagent participating in an oxidation-reduction reaction.

The present invention also relates to a method for manufacturing a sensor comprising:

the above-mentioned electrode; and
a reagent layer that is disposed in the first region and the second region of the electrode, and includes an outer peripheral edge portion positioned on the second outer peripheral edge portion of the second region, and a reagent participating in an oxidation-reduction reaction,
said method comprising:
coating the first region and the second region of the electrode with a first liquid composition containing the reagent in a first solvent, and then drying the first liquid composition to form the reagent layer.

The present invention also relates to a method for manufacturing a sensor comprising:

the above-mentioned electrode;
a reagent layer that is disposed in the first region and the second region of the electrode, and includes an outer peripheral edge portion positioned on the second outer peripheral edge portion of the second region, and a reagent participating in an oxidation-reduction reaction; and
a protective film that covers the reagent layer,
said method comprising:

coating the first region and the second region of the electrode with a first liquid composition including the reagent in a first solvent, and then drying the first liquid composition to form the reagent layer, and
coating the surface portion of the electrode with a second liquid composition containing a protective film component in a second solvent so as to cover the reagent layer, and then drying the second liquid composition to form the protective film.

A preferred embodiment of the present invention relates to an electrode to be disposed on an insulating substrate, said electrode comprising:

a surface portion that is positioned on the opposite side from the substrate when the electrode is disposed on the substrate;
a first region that is formed on the surface portion, includes a first outer peripheral edge portion, and at least part of which has a first surface free energy;
a second region that is formed on the surface portion, surrounds the first region, includes a second inner peripheral edge portion in contact with the first outer peripheral edge portion of the first region, and a second outer peripheral edge portion positioned more to the outside than the second inner peripheral edge portion, and has a second surface free energy that is greater than the first surface free energy;

a third region that is formed on the surface portion, surrounds the second region, includes a third inner peripheral edge portion in contact with the second outer peripheral edge portion of the second region, and a third outer peripheral edge portion positioned more to the outside than the third inner peripheral edge portion, and has a third surface free energy that is less than the second surface free energy;

a fourth region that is formed on the surface portion, surrounds the third region, includes a fourth inner peripheral edge portion in contact with the third outer peripheral edge portion of the third region, and a fourth outer peripheral edge portion positioned more to the outside than the fourth inner peripheral edge portion, and has a fourth surface free energy that is greater than the third surface free energy; and

an insulating layer, at least part of which is disposed on the surface portion, and that includes an opening formed in the first region, the second region, the third region, and the fourth region, having an inner peripheral edge portion in contact with the fourth outer peripheral edge portion of the fourth region in plan view from a direction perpendicular to the surface portion, and passing through in the thickness direction.

Another preferred embodiment of the present invention relates to a method for manufacturing the electrode including the surface portion, the first region, the second region, the third region, the fourth region, and the insulating layer, said method comprising the steps of:

preparing an untreated electrode comprising:

an untreated surface portion having an untreated region for forming the first region, the second region, the third region, and the fourth region in a part of the untreated surface portion, and having the first surface free energy, and

an insulating layer, at least part of which is disposed on the untreated surface portion, and that includes an opening formed in the untreated region and passing through in the thickness direction; and

irradiating, out of the untreated region of the untreated surface portion in the untreated electrode, an annular fourth untreated region disposed on the outer periphery of the untreated region adjacent to the inner peripheral edge portion of the opening in the insulating layer and an annular second untreated region disposed further toward the inside than the fourth untreated region in a plan view from a direction perpendicular to the untreated surface portion, with a laser beam, thereby converting the fourth untreated region into the fourth region, and converting the second untreated region into the second region.

In the electrode obtained by this method, preferably, the third surface free energy is the same as the first surface free energy, the first untreated region, which is the portion of the untreated region that is further to the inside than the second untreated region, becomes the first region having the first surface free energy, and the third untreated region, which is the portion of the untreated region between the second untreated region and the fourth untreated region, becomes the third region having the third surface free energy that is the same as the first surface free energy.

ADVANTAGEOUS EFFECTS

The electrode according to the present invention allows a reagent layer of the designed dimensions to be formed very accurately in the first and second regions formed on the surface portion of the electrode.

An analyte can be detected with the sensor according to the present invention.

With the method for manufacturing a sensor according to the present invention, a sensor having a reagent layer with the designed dimensions in a first region and a second region of an electrode can be manufactured efficiently.

In a preferred embodiment of the present invention, an electrode having a surface portion, a first region, a second region, a third region, a fourth region, and an insulating layer allows a reagent layer of the designed dimensions to be accurately formed in the first region and the second region, and allows a protective film covering the reagent layer, the third region, and the fourth region to be accurately formed.

In another preferred aspect of the present invention, a method for manufacturing an electrode having a surface portion, a first region, a second region, a third region, a fourth region, and an insulating layer makes it easy to form the first region, the second region, the third region, and the fourth region very accurately, each at its designed position.

BRIEF DESCRIPTION OF DRAWINGS

Fig. 1 is a plan view of a substrate on which two electrodes, a reference electrode conductive layer, a counter electrode, and wiring are disposed;

Fig. 2 is a plan view of a substrate on which a first region and a second region are formed on the surface portion of each of the two electrodes shown in Fig. 1;

Fig. 3 is a plan view of the substrate in Fig. 2 on which an insulating layer is further disposed;

Fig. 4 is a plan view of the substrate in Fig. 3 on which a reagent layer is further disposed on the electrodes;

Fig. 5 is a plan view of the sensor according to an embodiment of the present invention;

Fig. 6 is a schematic diagram illustrating a method for measuring an analyte by immersing the sensor shown in Fig. 5 in a liquid sample containing cells;

Fig. 7 is a detail plan view of the portion bounded by the broken line in Fig. 2, which includes the first region and the second region of the electrode according to an embodiment of the present invention;

Fig. 8 is a cross-sectional view along the D-D' line of the electrode shown in Fig. 7;

Fig. 9 is a cross-sectional view of a first liquid composition containing a reagent participating in a redox reaction in a first solvent, which is applied to the first region and the second region of the electrode shown in Fig. 8;

Fig. 10 is a cross-sectional view of the reagent layer disposed in the first and second regions of the electrode, which is formed by drying the first liquid composition shown in Fig. 9;

Fig. 11 is a cross-sectional view along the A-A' line of the portion of the sensor shown in Fig. 5 that includes the working electrode (including an electrode, a reagent layer, and a protective film);

Fig. 12 is a cross-sectional view along the B-B' line of the portion of the sensor shown in Fig. 5 that includes the reference electrode (including a reference electrode conductive layer, a silver/silver chloride layer, and a protective film);

Fig. 13 is a cross-sectional view along the C-C' line of the portion of the sensor shown in Fig. 5 that includes a counter electrode;

Fig. 14 is a plan view of the portion of an electrode according to another embodiment of the present invention that includes a first region and a second region;

Fig. 15 is a plan view of the portion of an electrode according to yet another embodiment of the present invention that includes a first region and a second region;

Fig. 16 is a plan view of the portion of an electrode according to yet another embodiment of the present invention that includes a first region and a second region;

Fig. 17 is a plan view of the portion of an electrode according to yet another embodiment of the present invention that includes a first region, a second region, a third region, and a fourth region;

Fig. 18 is a cross-sectional view along the E-E' line of the electrode shown in Fig. 17;

Fig. 19 is a cross-sectional view of a first liquid composition containing a reagent participating in a redox reaction in a first solvent, which is applied to the first region and the second region of the electrode shown in Fig. 18;

Fig. 20 is a cross-sectional view of a reagent layer disposed in the first and second regions of the electrode, which is formed by drying the first liquid composition shown in Fig. 19;

Fig. 21 is a cross-sectional view of a second liquid composition containing a protective film component in a second solvent, which is applied in the first region, the second region, the third region, and the fourth region of the electrode shown in Fig. 20, so as to cover the reagent layer;

Fig. 22 is a cross-sectional view of a protective film disposed in the first region, the second region, the third region, and the fourth region of the electrode, so as to cover the reagent layer, in which the protective film is formed by drying the second liquid composition shown in Fig. 21;

Fig. 23 is a plan view of the portion of an electrode according to yet another embodiment of the present invention that includes an opening in an insulating layer, a first region, and a second region;

Fig. 24 is a cross-sectional view along the F-F' line of the electrode shown in Fig. 23;

Fig. 25 is a cross-sectional view of a sensor according to an embodiment of the present invention, in which a reagent layer and a protective film are disposed in the first region and the second region within the opening in the insulating layer of the electrode shown in Fig. 23;

Fig. 26 is a control block diagram of an analysis device including a sensor according to an embodiment of the present invention;

Fig. 27 is a photograph showing the results of Experiment 1;

Fig. 28 is a photograph showing the results of Experiment 3;

Fig. 29A shows the results of continuous measurement over 12 days of the current value of a sensor in which 0.40 mg of a lactate oxidase-containing liquid composition used to prepare a reagent layer was applied in Experiment 5;

Fig. 29B shows the results of continuous measurement over 12 days of the current value of a sensor in which 0.45 mg of a lactate oxidase-containing liquid composition used to prepare a reagent layer was applied in Experiment 5;

Fig. 29C shows the results of continuous measurement over 12 days of current value of a sensor in which 0.50 mg of a lactate oxidase-containing liquid composition used to prepare a reagent layer was applied in Experiment 5;

Fig. 29D shows the results of continuous measurement over 12 days of current value of a sensor in which 0.55 mg of a lactate oxidase-containing liquid composition used to prepare a reagent layer was applied in Experiment 5;

Fig. 29E shows the results of continuous measurement over 12 days of the current value of a sensor in which 0.60 mg of a lactate oxidase-containing liquid composition used to prepare a reagent layer was applied in Experiment 5;

Fig. 30 is a plan view of the portion of an electrode according to yet another embodiment of the present invention that includes an opening in an insulating layer, and a first region, a second region, a third region, and a fourth region;

Fig. 31 is a cross-sectional view along the G-G' line of the electrode shown in Fig. 30;

Fig. 32 is a cross-sectional view of a working electrode (sensor) according to an embodiment of the present invention, in which a reagent layer is disposed in the first region and the second region within the opening of the insulating layer of the electrode shown in Fig. 30, and a protective film is disposed on the reagent layer and in the third region and the fourth region;

Fig. 33 is a cross-sectional view of an untreated electrode for manufacturing the electrode shown in Fig. 30;

Fig. 34 is a schematic diagram of the portion of the untreated electrode shown in Fig. 33 that includes the boundary between the untreated region of the untreated surface portion and the insulating layer around the opening, in a plan view perpendicular to the untreated surface portion;

Fig. 35 is a schematic diagram of a portion of an electrode obtained by converting a fourth untreated region of the untreated surface portion of the untreated electrode shown in Fig. 34 into a fourth region, and converting a second untreated region of the untreated region of the untreated surface portion into a second region;

Fig. 36 is a schematic diagram of a portion of an electrode obtained by converting the portion of the untreated surface portion exposed by using laser beam irradiation to break up the region around the opening in the insulating layer into a fourth region in addition to the fourth untreated region of the untreated region of the untreated surface portion in the untreated electrode shown in Fig. 34, and converting the second untreated region of the untreated region of the untreated surface portion into a second region;

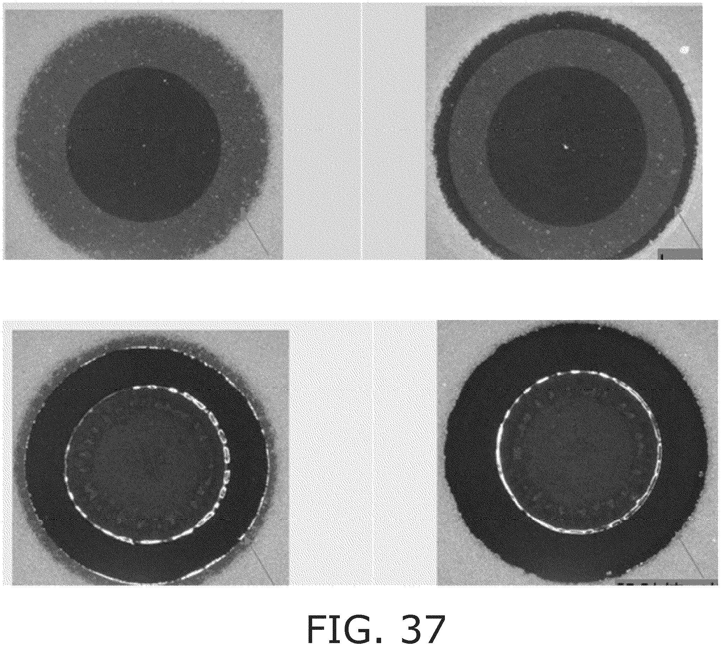

in Fig. 37, the upper left photograph shows the electrode prepared in Experiment 6-2 (including a circular second region and a third region surrounding this, within the opening of the insulating layer) after laser beam irradiation, the lower left photograph shows the working electrode obtained by forming a reagent layer and a protective film on the electrode prepared in Experiment 6-2, the upper right photograph shows the electrode prepared in Experiment 6-1 (including a circular second region and a third region surrounding this, and a fourth region surrounding this, within the opening of the insulating layer) after laser beam irradiation, and the lower right photograph shows the working electrode obtained by forming a reagent layer and a protective film on the electrode prepared in Experiment 6-1; and

in Fig. 38, the part on the left shows the results of measuring the thickness of the protective film on the working electrode obtained by forming a reagent layer and a protective film on the electrode prepared in Experiment 6-2, and the part on the right shows the results of measuring the thickness of the protective film on the working electrode obtained by forming a reagent layer and a protective film on the electrode prepared in Experiment 6-1.

DESCRIPTION OF EMBODIMENTS

Embodiments of the electrode, sensor, and method for manufacturing a sensor according to the present invention will now be described. In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand this disclosure, but does not intend for these to limit what are claimed.

This specification encompasses the disclosure of Japanese Patent Application No. 2022-124198, which is the priority document of this application.

Also, all publications, patents, and patent applications cited herein are hereby incorporated by reference in their entirety.

Materials

Examples of materials that can be used in the electrodes and sensors disclosed herein are given below.

There are no particular limitations on the material of the insulating substrate on which the electrodes disclosed in the present specification are disposed, but examples include polyethylene terephthalate, polycarbonate, polyimide, polyethylene, polypropylene, polystyrene, polyvinyl chloride, polyoxymethylene, monomer cast nylon, cycloolefin polymer, polyethylene naphthalate, polybutylene terephthalate, methacrylic resin, ABS resin, and other such resin materials, and a glass material can also be used. Polyethylene terephthalate, polycarbonate, and polyimide are favorable, and polyethylene terephthalate is especially favorable. There are no particular limitations on the thickness and other such dimensions of the substrate, but the substrate thickness may be, for example, at least 0.05 mm and no more than 2 mm, with at least 0.1 mm and no more than 1 mm being preferable.

The electrode disclosed in this specification can be formed from a conductive material such as carbon, gold, platinum, or palladium. The carbon can be a conductive carbon material such as glassy carbon, carbon black, graphite, diamond-like carbon, graphene, carbon nanotubes, or fullerene. The electrode disclosed in this specification is preferably a layer of a conductive material (conductive layer). The conductive layer can be formed from one of the conductive materials mentioned above, by sputtering, vapor deposition, screen printing, or another such method. The conductive layer can be worked into a specific pattern by laser trimming as needed.

The reference electrode conductive layer, counter electrode, and wiring comprised in the sensor disclosed in this specification can also be made of the same conductive materials as discussed above.

The electrode and sensor disclosed herein may include an insulating layer. The insulating layer preferably has a water-repellent surface. Here, the term "water-repellent surface" refers to a surface having a contact angle with water of, for example, 90° or more, preferably 100° or more, and most preferably 110° or more. There are no particular limitations on the upper limit of the water contact angle of the water-repellent surface of the insulating layer, but this angle may be, for example, 160° or less. That is, the water contact angle of the water-repellent surface of the insulating layer is, for example, 90° or more and 160° or less, and preferably falls within a narrower range defined by the upper limit and/or lower limit value. The water contact angle of the surface of the insulating layer can be the measured value at 20°C. The water contact angle of the surface of the insulating layer can be measured with a commercially available analysis apparatus. For example, a Handy Contact Angle and Surface Free Energy Analyzer MSA manufactured by Krüss. The water contact angle of the surface is preferably measured by discharging a 1-μL droplet of water onto the surface to be measured, and measuring the contact angle between the droplet and the surface after 2 seconds.

The insulating layer of the electrode and the sensor disclosed in this specification preferably contains a fluororesin. This fluororesin can be a polymer compound of a fluorohydrocarbon, such as a polymer compound containing one or more from among vinylidene fluoride, tetrafluoroethylene, hexafluoropropylene, and perfluoro(alkyl vinyl ether), with a copolymer containing two or more selected from vinylidene fluoride, tetrafluoroethylene, hexafluoropropylene, and perfluoro(alkyl vinyl ether) being particularly favorable, and a copolymer containing vinylidene fluoride and hexafluoropropylene being better yet. The entire insulating layer can be made of a fluororesin.

Another preferred example of the insulating layer comprised in the electrode and sensor disclosed in this specification is an insulating layer containing a compound that includes a perfluoroalkyl group. An example of a compound including a perfluoroalkyl group is a fluorine-based surface-modifying additive. The insulating layer containing a compound that includes a perfluoroalkyl group can be formed by applying a composition containing an insulating matrix resin and a compound including a perfluoroalkyl group (fluorine-based surface-modifying additive) in a solvent, and then drying the composition applied. The insulating layer thus formed is preferably a layer of an insulating matrix resin containing a compound that includes a perfluoroalkyl group on the surface. There are no particular limitations on the type of insulating matrix resin, but this resin may be a polyester resin, for example. There are no particular limitations on the number of carbon atoms of the perfluoroalkyl group, but this number can range between 2 and 20 carbon atoms, for example. The perfluoroalkyl group may contain a trifluoromethyl group at the end.

The insulating layer may have a single-layer structure consisting of just one type of insulating layer, or may have a laminate structure in which two or more insulating layers are laminated.

The sensor disclosed in this specification is immersed in a liquid sample and used to detect a specific analyte in the liquid sample. The liquid sample is preferably contains water as a solvent. Examples of the liquid sample include a cell culture medium and a liquid sample prepared using blood collected from a living organism. Examples of the analyte include glucose, lactic acid, cholesterol, bilirubin, amino acids such as glutamine and glutamic acid, glycated amino acids, glycated peptides, ketone bodies (3-hydroxybutyric acid), and alcohol.

The sensor disclosed in this specification has a working electrode including the electrode disclosed in this specification and a reagent layer containing a reagent that participates in an oxidation-reduction reaction. The reagent that participates in the oxidation-reduction reaction may be any reagent that participates in an oxidation-reduction reaction with the analyte, and can be appropriately selected depending on the analyte. The reagent that participates in the oxidation-reduction reaction may include a combination of an oxidoreductase and a mediator (electron transfer substance), or just an oxidoreductase. The oxidoreductase may include a coenzyme.

Examples of oxidoreductase include oxidase and dehydrogenase. Specific examples of the oxidoreductase include glucose oxidase, lactate oxidase, cholesterol oxidase, bilirubin oxidase, glucose dehydrogenase, lactate dehydrogenase, amino acid oxidase, amino acid dehydrogenase, glutamate oxidase, glutamate dehydrogenase, fructosyl amino acid oxidase, fructosyl peptide oxidase, 3-hydroxybutyrate dehydrogenase, alcohol oxidase, and alcohol dehydrogenase. These oxidoreductases can be used to detect the analytes discussed above.

There are no particular limitations on the mediator, which can be one or more types selected from among metal complexes (such as osmium complexes, ruthenium complexes, iron complexes, etc.), quinone compounds (such as benzoquinone, naphthoquinone, phenanthrenequinone, phenanthrolinequinone, anthraquinone, and derivatives of these), phenazine compounds, viologen compounds, phenothiazine compounds, and phenol compounds. Specific examples of the mediator include one or more types selected from among potassium ferricyanide, hexaammineruthenium, ferrocene, poly(1-vinylimidazole)-bis(bipyridine)chloroosmium, hydroquinone, 2-methyl-1,4-benzoquinone, a 1,2-naphthoquinone-4-sulfonic acid salt, a 9,10-phenanthrenequinone-2-sulfonic acid salt , a 9,10-phenanthrenequinone-2,7-disulfonic acid salt, 1,10-phenanthroline-5,6-dione, a anthraquinone-2-sulfonic acid salt, phenazine derivatives (1-methoxy-5-methylphenazinium methyl sulfate, 1-methoxy-5-ethylphenazinium ethyl sulfate, etc.), methyl viologen, benzyl viologen, methylene blue, methylene green, 2-aminophenol, 2-amino-4-methylphenol, and 2,4-diaminophenol. There are no particular limitations on the above-mentioned salt, but examples include sodium salts, potassium salts, calcium salts, magnesium salts, and lithium salts.

From the standpoints of durability of the sensor and preventing the mediator from flowing out of the sensor, the mediator is preferably a mediator bound to a polymer compound, which is referred to as a high molecular mediator. The polymer compound to which the mediator is bound can be a homopolymer, a random copolymer, a block copolymer, or a polymer compound in which these are bound or mixed. The weight-average molecular weight of the polymer compound is, for example, 10,000 or more, preferably 50,000 or more, and more preferably 100,000 or more, and the upper limit to the weight-average molecular weight is, for example, less than 10,000,000, and preferably less than 1,000,000. That is, the weight-average molecular weight of the polymer compound can be 10,000 or more and less than 10,000,000, and preferably falls within a narrower range defined by the upper limit and/or lower limit value. There are no particular limitations on the polymer compound, but examples include those in which a plurality of at least one type of atom selected from among carbon atoms, nitrogen atoms, oxygen atoms, and sulfur atoms are bound in a chain to constitute a main chain. Specific examples thereof include natural polymer compounds such as proteins, polypeptides, and polynucleotides, and synthetic polymer compounds such as polyamino acids, polyimines, polyallyl compounds, poly(meth)acrylates, polyalkylene oxides, and copolymers of these. Examples of polyamino acids include poly(L-glutamic acid) and poly(L-lysine). Examples of polyimines include polyalkyleneimines such as polyethyleneimine or polypropyleneimine. Examples of polyallyl compounds include polyallylamine and polydiallylamine. Examples of polyalkylene oxides include polyethylene oxide and polypropylene oxide. The high molecular mediator is preferably hydrophilic as a whole, and it is even more preferable for the polymeric compound to which the mediator is bonded to be hydrophilic.

The reagent layer of the sensor disclosed in this specification may further contain, in addition to the reagent, components such as a buffer, a hydrophilic polymer compound, a conductive carbon filler, and a crosslinking agent. An example of a hydrophilic polymer compound is a cellulose derivative, and the cellulose derivative may be one or more types selected from among methyl cellulose, carboxymethyl cellulose, hydroxyethyl cellulose, hydroxypropyl cellulose, hydroxyethyl methyl cellulose, and hydroxypropyl methyl cellulose. The conductive carbon filler may be one or more types selected from among carbon black, graphite powder, porous carbon, and nanocarbon.

The reagent layer of the sensor disclosed in this specification can be formed by applying a first liquid composition containing a reagent that participates in an oxidation-reduction reaction in a first solvent, and drying the first liquid composition applied. The first liquid composition may further contain the above-mentioned components described as components contained in the reagent layer. The first solvent contained in the first liquid composition can be any solvent capable of dissolving the reagent, an example of which is a solvent containing water and/or an alcohol, with one or more types selected from among water and alcohol being preferable, with water or a mixed solvent of water and alcohol being more preferable, and with water being the most preferable. Examples of the alcohol include monohydric alcohols having 1 to 5 carbon atoms, with methanol, ethanol, and isopropyl alcohol being particularly favorable, and with ethanol the most favorable .

The sensor disclosed in this specification may further include a protective film that covers the reagent layer. The protective film can be any film that prevents or inhibits leakage of the reagent contained in the reagent layer to the outside of the protective film, and allows the analyte present outside the protective film to permeate. A protective film having such properties preferably contains a polymer compound. Examples of the polymer compound contained in the protective film include a polymer compound containing 4-vinylpyridine as a structural unit, and a polymer compound containing a cation exchange functional group such as a proton conductive group.

Examples of polymer compounds containing 4-vinylpyridine as a structural unit include poly(4-vinylpyridine), a copolymer (preferably a block copolymer) of 4-vinylpyridine and a methacrylic acid alkyl ester, and a copolymer (preferably a random copolymer) of styrene, 4-vinylpyridine, and oligopropylene glycol methyl ether methacrylate. An example of a methacrylic acid alkyl ester is tert-butyl methacrylate. An example of oligopropylene glycol methyl ether methacrylate include tripropylene glycol methyl ether methacrylate. The polymer compound containing 4-vinylpyridine as a structural unit is preferably crosslinked with a crosslinking agent such as polyethylene glycol diglycidyl ether (PEGDGE). This crosslinking agent can be one that contains two or more epoxy groups such as PEGDGE. The polymer compound containing 4-vinylpyridine as a structural unit and crosslinked with a crosslinking agent containing two or more epoxy groups includes a quaternary ammonium cation-containing functional group produced by the reaction of a pyridyl group (tertiary amine) derived from 4-vinylpyridine with an epoxy group.

Examples of a polymer compound containing a cation exchange functional group such as a proton conductive group include polymer compounds containing a structural unit having a sulfonic acid group on a side chain, with a polymer compound containing a perfluoro compound having a sulfonic acid group on a side chain as a structural unit being preferable, a copolymer compound containing a perfluoro compound having a sulfonic acid group on a side chain and a perfluoro compound having no ionic functional group on a side chain as a structural unit being more preferable, a copolymer of tetrafluoroethylene and perfluoro[2-(fluorosulfonylethoxy)propylvinylether] being particularly favorable, and Nafion (registered trademark) being especially good. The protective film containing a polymer compound that includes a cation exchange functional group is preferably provided on the reagent layer in order to transport cations such as protons between the reagent layer and the liquid sample.

The protective film can be a laminate of two or more protective films, such as a laminate of a protective film containing a polymer compound that includes a cation exchange functional group, which is provided on the side in contact with the reagent layer containing a reagent that generates protons through an oxidation-reduction reaction of the analyte, and a protective film containing a polymer compound containing 4-vinylpyridine as a structural unit, which is provided over the first film.

Another favorable example of a protective film is one that contains a polymer compound including 4-vinylpyridine as a structural unit.

The reference electrode of the sensor disclosed in this specification may have a protective film. The protective film of the reference electrode may be made of the materials described above for the protective film of the working electrode.

The protective film of the sensor disclosed in this specification can be formed by applying a second liquid composition containing protective film components in a second solvent, and then drying the second liquid composition applied. Examples of the second solvent include alcohol and a mixed solvent of alcohol and water. Examples of the alcohol include monohydric alcohols having 1 to 5 carbon atoms, with methanol, ethanol, and isopropyl alcohol being particularly favorable, and with ethanol being the most favorable. The protective film components are components that can form a protective film after drying. The protective film components are, for example, one or more types selected from among polymer compounds that form a protective film, their monomers, and their prepolymers, and may further include a crosslinking agent.

Surface Free Energy

In this specification, the "surface free energy" of a specific location of the surface portion of an electrode is the surface free energy found by the Owens-Wendt method. This surface free energy $\gamma s$ (mN/m) is the sum of the polar component $\gamma sp$ (mN/m) and the dispersive component $\gamma sd$ (mN/m).

More specifically, the polar component $\gamma sp$ (mN/m), the dispersive component $\gamma sd$ (mN/m), and the surface free energy $\gamma s$ (mN/m) can be obtained by using a contact angle meter to measure the contact angles (drop amount of 1 $\mu L$, measured 2 seconds after dropping) of water and diiodomethane at a specific location on the surface portion of the electrode at 20°C and a relative humidity of 40%, and then calculating from the simultaneous equations consisting of the following formulas (1), (2) and (3).

$$\text{Formula (1): } \gamma s = \gamma sd + \gamma sp$$

$$\text{Formula (2): } 72.8(1 + \cos\theta H) = 2(21.8\gamma sd)^{1/2} + 2(51.0\gamma sp)^{1/2}$$

$$\text{Formula (3): } 50.8(1 + \cos\theta I) = 2(48.5\gamma sd)^{1/2} + 2(2.3\gamma sp)^{1/2}$$

$\gamma s$: surface free energy
$\gamma sd$: dispersive component of surface free energy
$\gamma sp$: polar component of surface free energy
$\theta H$: contact angle with water
$\theta I$: contact angle with diiodomethane

The contact angles with respect to water and diiodomethane at a specific location of the surface portion of the electrode can be measured using a commercially available analyzer, such as a Handy Contact Angle and Surface Free Energy Analyzer MSA manufactured by Krüss. The contact angles with respect to water and diiodomethane at a specific location of the surface portion of the electrode are preferably measured by placing 1-$\mu L$ droplets of water and diiodomethane onto the surface of the measurement region at 20°C and a relative humidity of 40%, and measuring the contact angles between the droplets and the surface after 2 seconds.

In this specification, the second surface free energy is greater than the first surface free energy, although there are no particular limitations on the difference between the two. For example, the second surface free energy is preferably at least 3.0 mN/m, more preferably at least 5.0 mN/m, even more preferably at least 7.0 mN/m, still more preferably at least 10.0 mN/m, more preferably yet at least 12.0 mN/m, and most preferably at least 15.0 mN/m greater than the first surface free energy. There is no particular upper limit to the difference between the second surface free energy and the first surface free energy, but the difference can be, for example, 50 mN/m or less, and preferably 30 mN/m or less. That is, the difference between the second surface free energy and the first surface free energy can be, for example, at least 3.0 mN/m and no more than 50 mN/m, and preferably falls within a narrower range defined by the upper limit and/or lower limit value. In the case described in this paragraph, the region of the surface portion of the electrode having a first surface free energy has lower wettability and the region having a second surface free energy has higher wettability by the first liquid composition containing a reagent that participates in an oxidation-reduction reaction in a first solvent, which achieves the effect described below, that of being able to form a reagent layer of stable dimensions on the first and second regions of an electrode.

In particular, it is preferable for the polar component of the second surface free energy to be greater than the polar component of the first surface free energy. For example, the polar component of the second surface free energy is preferably at least 2.0 mN/m, more preferably at least 3.0 mN/m, even more preferably at least 5.0 mN/m, better yet at least 8.0 mN/m, and most preferably at least 11.0 mN/m greater than the polar component of the first surface free energy. There is no particular upper limit to the difference between the polar component of the second surface free energy and the polar component of the first surface free energy, but the difference can be, for example, 40 mN/m or less, and preferably 25 mN/m or less. That is, the difference between the polar component of the second surface free energy and the polar component of the first surface free energy can be, for example, at least 2.0 mN/m and no more than 40 mN/m, and preferably falls within a narrower range defined by the upper limit and/or lower limit value. In the case described in this paragraph, the region of the surface portion of the electrode having a first surface free energy has lower wettability and the region having a second surface free energy has higher wettability by the first liquid composition containing a reagent that participates in an oxidation-reduction reaction in a first solvent, so the effect described below of being able to form a reagent layer with stable dimensions on the first and second regions of the electrode is particularly pronounced.

There are no particular limitations on the value of the first surface free energy, but may be, for example, at least 20.0 mN/m, preferably at least 30.0 mN/m, more preferably at least 40.0 mN/m, and particularly preferably at least 45.0 mN/m, and may be, for example, 70.0 mN/m or less, preferably 60.0 mN/m or less, and more preferably 50.0 mN/m or less. That is, the value of the first surface free energy may be, for example, at least 20.0 mN/m and no more than 70.0 mN/m, and preferably, may fall within a narrower range defined by the upper limit and/or lower limit value. There are no particular limitations on the value of the polar component of the first surface free energy, but this value may be, for example, 5.0 mN/m or less, and preferably 3.0 mN/m or less, and is, for example, at least 0.0 mN/m, and preferably at least 2.0 mN/m. That is, the value of the polar component of the first surface free energy is at least 0.0 mN/m and no more than 5.0 mN/m, and preferably falls within a narrower range defined by the upper limit and/or lower limit value. The ratio of the dispersive component to the polar component ($\gamma sd/\gamma sp$) of the first surface free energy can be, for example, 8.0 or more, preferably 10.0 or more, more preferably 12.0 or more, particularly preferably 15.0 or more, and can be, for example, 50.0 or less, and preferably 30.0 or less. That is, the ratio of the dispersive component to the polar component of the first surface free energy can be, for example, 8.0 or more and 50.0 or less, and preferably falls within a narrower range defined by the upper limit and/or lower limit value. The region of the surface portion of the electrode having the first surface free energy described in this paragraph has good liquid repellency and low wettability with respect to water and alcohol, and especially to water. A carbon electrode is an example of an electrode having a surface portion with the first surface free energy described in this paragraph prior to the treatment to increase the surface free energy discussed below.

There are no particular limitations on the value of the second surface free energy, but this value is, for example, at least 45.0 mN/m, preferably at least 50.0 mN/m, more preferably at least 55.0 mN/m, even more preferably at least 59.0 mN/m, and particularly preferably at least 60.0 mN/m, and can be, for example, 100.0 mN/m or less, preferably 80.0 mN/m or less, and more preferably 70.0 mN/m or less. That is, the value of the second surface free energy is, for example, at least 45.0 mN/m and no more than 100.0 mN/m, and preferably falls within a narrower range specified by the upper limit and/or lower limit value. There are no particular limitations on the value of the polar component of the second surface free energy, but this value may be, for example, at least 5.0 mN/m, preferably at least 8.0 mN/m, more preferably at least 10.0 mN/m, and particularly preferably at least 14.0 mN/m, and may be, for example, 50.0 mN/m or less, preferably 30.0 mN/m or less, and more preferably 20.0 mN/m or less. That is, the value of the polar component of the second surface free energy may be, for example, at least 5.0 mN/m and no more than 50.0 mN/m, and preferably falls within a narrower range defined by the upper limit and/or lower limit value. The ratio of the dispersive component to the polar component ($\gamma sd/\gamma sp$) of the second surface free energy may be, for example, 10.0 or less, preferably 8.0 or less, more preferably 6.0 or less, and particularly preferably 5.0 or less, and may be, for example, 1.0 or more. That is, the ratio of the dispersive component to the polar component of the second surface free energy is, for example, at least 1.0 and no more than 10.0, and preferably falls within a narrower range defined by the upper and/or lower limit value. The region of the surface portion of the electrode having the second surface free energy described in this paragraph has low liquid repellency and high wettability with respect to water and alcohol, especially to water.

In this specification, the third surface free energy is less than the second surface free energy. In other words, the second surface free energy is greater than the third surface free energy, and the specific difference can be selected from the same range as the difference described for the difference between the second surface free energy and the first surface free energy. In particular, it is preferable for the polar component of the second surface free energy to be greater than the polar component of the third surface free energy, and the specific value of this difference can be selected from the same range as the value described for the difference between the polar component of the second surface free energy and the polar component of the first surface free energy. The value of the third surface free energy, the value of the polar component of the third surface free energy, and the ratio of the dispersive component to the polar component ($\gamma sd/\gamma sp$) of the third surface free energy can be selected from the ranges described for the value of the first surface free energy, the value of the polar component of the first surface free energy, and the ratio of the dispersive component to the polar component of the first surface free energy, respectively, and are particularly preferably the same as the value of the first surface free energy, the value of the polar component of the first surface free energy, and the ratio of the dispersive component to the polar component of the first surface free energy, respectively.

In this specification, the fourth surface free energy is greater than the third surface free energy. The fourth surface free energy may be the same as or different from the second surface free energy. The specific difference between the fourth surface free energy and the third surface free energy can be selected from the same range as that described for the difference between the second surface free energy and the first surface free energy. In particular, it is preferable for the polar component of the fourth surface free energy to be greater than the polar component of the third surface free energy, and the specific value of the difference can be selected from the same range as that described for the difference between the polar component of the second surface free energy and the polar component of the first surface free energy. The value of the fourth surface free energy, the value of the polar component of the fourth surface free energy, and the ratio of the dispersive component to the polar component ($\gamma sd/\gamma sp$) of the fourth surface free energy can be selected from the ranges described for the value of the second surface free energy, the value of the polar component of the second surface free energy, and the ratio of the dispersive component to the polar component of the second surface free energy, respectively, and are particularly preferably the same as the value of the second surface free energy, the value of the polar component of the second surface free energy, and the ratio of the dispersive component to the polar component of the second surface free energy, respectively.

In this specification, the fifth surface free energy is less than the fourth surface free energy. In other words, the fourth surface free energy is greater than the fifth surface free energy, and the specific difference between them can be selected from the same range as the difference between the second surface free energy and the first surface free energy. In particular, it is preferable for the polar component of the fourth surface free energy to be greater than the polar component of the fifth surface free energy, and the specific value of the difference between them can be selected from the same range as that between the polar component of the second surface free energy and the polar component of the first surface free energy. The value of the fifth surface free energy, the value of the polar component of the fifth surface free energy, and the ratio of the dispersive component to the polar component ($\gamma sd/\gamma sp$) of the fifth surface free energy can be selected from the value of the first surface free energy, the value of the polar component of the first surface free energy, and the ratio of the dispersive component to the polar component of the first surface free energy, respectively, and are particularly preferably the same as the value of the first surface free energy, the value of the polar component of the first surface free energy, and the ratio of the dispersive component to the polar component of the first surface free energy, respectively.

In this specification, examples of the treatment for increasing the surface free energy of a specific region of the surface portion of the electrode include laser treatment, plasma treatment, corona discharge treatment, mask exposure, etc., with laser treatment being particularly favorable. These treatments are particularly preferable because they allow a region of the designed dimensions (outer shape and surface area) to be formed with higher accuracy than printing and other such methods. Laser working is a treatment for increasing the surface free energy of the specific region of the surface portion of the electrode by scanning and irradiating the region with a laser beam. This laser beam can be an ultraviolet (UV) laser or an SHG (second harmonic generation) laser. A UV laser usually has a wavelength of 355 nm. Favorable examples of treatment conditions using a UV laser are as discussed in the working examples. An SHG laser has a wavelength of 532 nm, for example, and has a green color in the visible light region. Examples of working conditions using a SHG laser include a wavelength of 532 nm, a laser output of 450 mW, a scanning rate of 2000 mm/s, and a frequency of 15 kHz.

Electrode 10

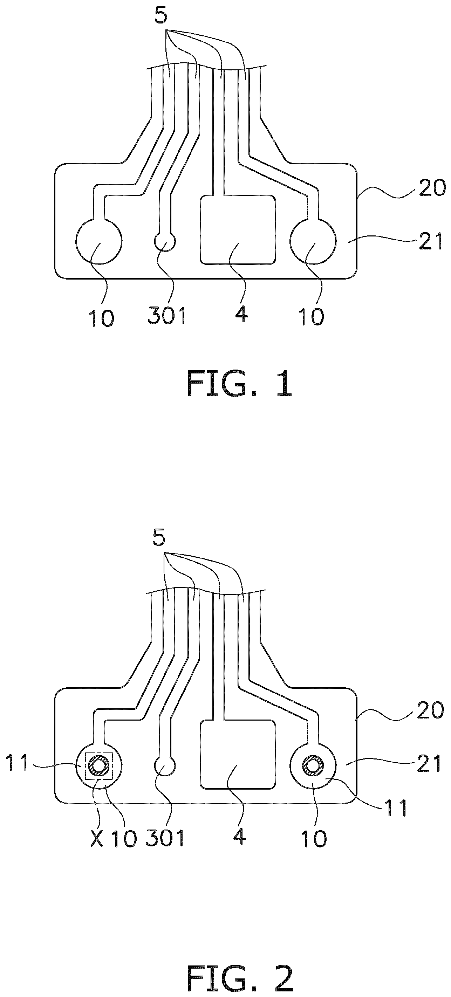

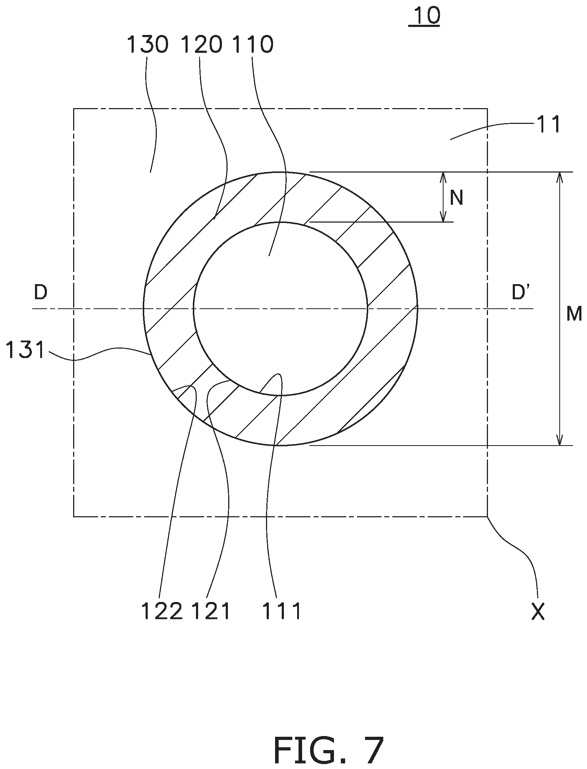

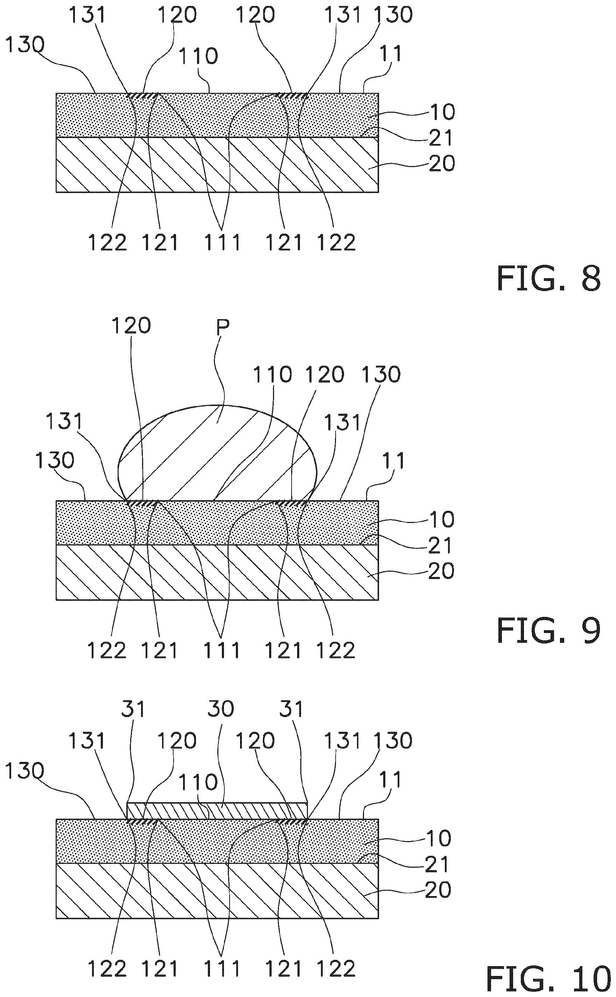

The electrode 10 according to an embodiment of the present invention will be described with reference to Figs. 2, 7, and 8. Fig. 7 is a detail view of the region X in Fig. 2, and Fig. 8 is a cross-sectional view along the D-D' line in Fig. 7.

The electrode 10 is disposed on an insulating substrate 20. In Figs. 2, 7, and 8, the electrode 10 is shown disposed on a first surface 21 of the substrate 20.

The electrode 10 includes a surface portion 11 positioned on the opposite side from the substrate 20 when disposed on the substrate 20, and a first region 110 and a second region 120 formed on the surface portion 11.

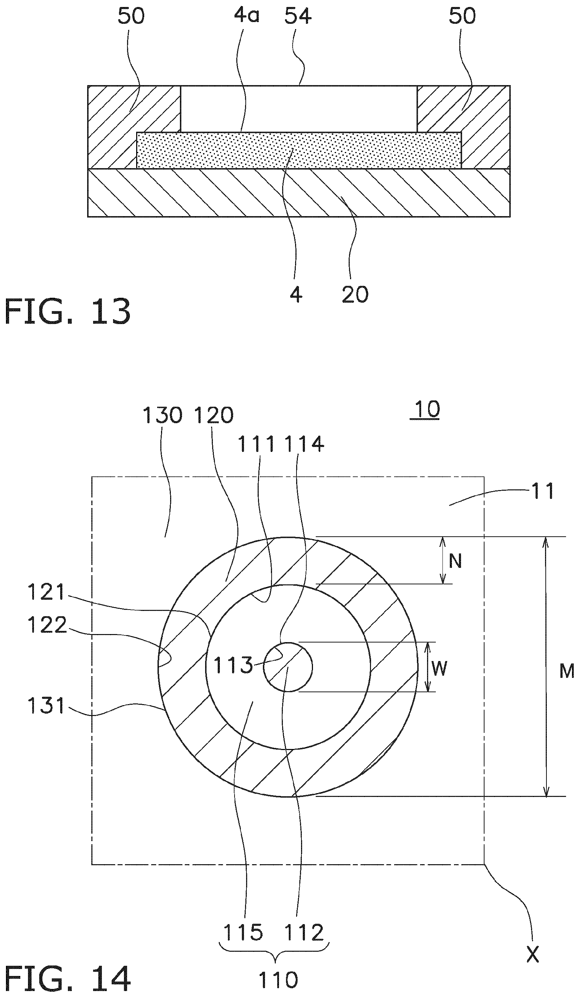

The first region 110 includes a first outer peripheral edge portion 111. At least part of the first region 110 has a first surface free energy. With the electrode 10 according to the embodiment shown in Figs. 2, 7, and 8, the entire first region 110 surrounded by the second region 120 has a first surface free energy. The part of the first region 110 having the first surface free energy is not limited to the example shown in Figs. 2, 7, and 8. For example, in the first region 110, all or a part of the first outer peripheral edge portion 111 in the circumferential direction can have the first surface free energy, and preferably, all or a part of the first outer peripheral edge portion 111 in the circumferential direction and the portion adjacent to this on the inside can have the first surface free energy. Other preferred embodiments of the first region 110 will be described below with reference to Figs. 14, 15, 16, etc.

The second region 120 is formed on the surface portion 11 and is a region surrounding the first region 110. The second region 120 includes a second inner peripheral edge portion 121 in contact with the first outer peripheral edge portion 111 of the first region 110, and a second outer peripheral edge portion 122 that is positioned more to the outside than the second inner peripheral edge portion 121. The second region 120 has a second surface free energy that is greater than the first surface free energy.

The electrode 10 according to the embodiment shown in Figs. 2, 7, and 8 further has a third region 130 that is formed on the surface portion 11, surrounds the second region 120, includes a third inner peripheral edge portion 131 in contact with the second outer peripheral edge portion 122 of the second region 120, and has a third surface free energy that is less than the second surface free energy.

The electrode 10 according to the embodiment shown in Figs. 2, 7, and 8 can be manufactured by selectively subjecting the part of the surface portion 11 having the first surface free energy of the electrode 10 that surrounds the first region 110 (see Figs. 7 and 8) to the treatment for increasing the surface free energy described in the "Surface Free Energy" section, thereby forming the second region 120 having a second surface free energy that is greater than the first surface free energy. At this point, the outside of the second region 120 becomes the third region 130 having a third surface free energy that is equal to the first surface free energy. In this embodiment, the entire first region 110 surrounded by the second region 120 has the first surface free energy.

In this embodiment, the first surface free energy, the second surface free energy, and the third surface free energy are each as described in the "Surface Free Energy" section.

The use of the electrode 10 having the surface portion 11, the first region 110, the second region 120 and the third region 130 according to the embodiment shown in Figs. 2, 7, and 8 has the effect that the reagent layer 30 of the designed dimensions can be formed very accurately in the first region 110 and the second region 120. The contact surface area between the reagent layer 30 and the electrode 10 is defined by the first region 110 and the second region 120 of the electrode 10. This effect will be described with reference to Figs. 9 and 10.

As shown in Fig. 9, the first region 110 and the second region 120 of the electrode 10 are coated with a first liquid composition P containing a reagent that participates in an oxidation-reduction reaction in a first solvent. At this point, the electrode 10 may have already been disposed on the first surface 21 of the substrate 20. The first liquid composition P has a relatively low wettability with respect to the first region 110 having a first surface free energy, a relatively high wettability with respect to the second region 120 having a second surface free energy, and a relatively low wettability with respect to the third region 130 having a third surface free energy. Therefore, the first liquid composition P applied to the first region 110 and the second region 120 rises up as it attempts to collected in the first region 110 in the center, forming a droplet whose outer periphery is defined by the second outer peripheral edge portion 122 of the second region 120. The first liquid composition P coating the first region 110 and the second region 120 of the electrode 10 is prevented from spreading outwardly by the third inner peripheral edge portion 131 of the third region 130. The first region 110 and the second region 120 of the electrode 10 can hold a larger amount of the first liquid composition P as a droplet than a solid circular region having a second surface free energy formed as an island in a region having a first surface free energy of the surface portion of the electrode as in the comparative example discussed below, which allows a reagent layer 30 containing a larger amount of reagent to be formed. The larger is the amount of reagent held in the reagent layer 30, the longer it will be possible to perform continuous measurement of the analyte (see Experiment 5).

The first liquid composition P coating the first region 110 and the second region 120 of the electrode 10 is then dried to form the reagent layer 30 including the reagent and an outer peripheral edge portion 31 located on the second outer peripheral edge portion 122 of the second region 120, which is disposed in the first region 110 and the second region 120 of the electrode 10 as shown in Fig. 10. As shown in Fig. 9, the outer periphery of the first liquid composition P coating the first region 110 and the second region 120 of the electrode 10 is defined by the second outer peripheral edge 122 of the second region 120, so the outer peripheral edge portion 31 of the reagent layer 30 obtained by drying is also located on the second outer peripheral edge portion 122 of the second region 120, as shown in Fig. 10. Accordingly, the outer peripheral shape and surface area of the reagent layer 30 can be adjusted by adjusting the outer shape and surface area of the first region 110 and the second region 120. Meanwhile, the thickness of the reagent layer 30 can be adjusted by adjusting the concentration of the reagent in the first liquid composition P and the amount of the first liquid composition P applied. That is, with the electrode 10 of this embodiment, the reagent layer 30 of the designed dimensions can be formed very accurately in the first region 110 and the second region 120 formed on the surface portion 11 of the electrode 10. The contact surface area between the reagent layer 30 and the electrode 10 is defined by the first region 110 and the second region 120 of the electrode 10.

The first solvent contained in the first liquid composition P can be any solvent capable of dissolving the reagent, examples of which include a solvent containing water and/or alcohol. Preferred examples of the first solvent are as described in the "Materials" section.

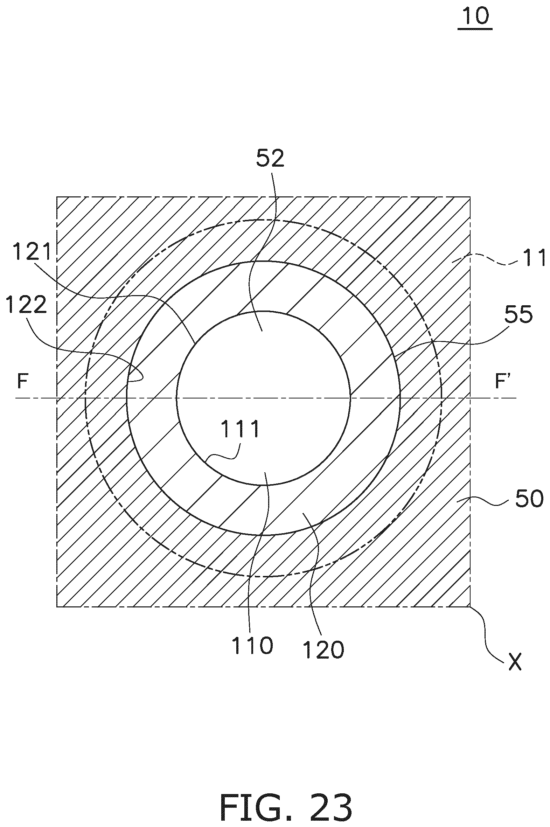

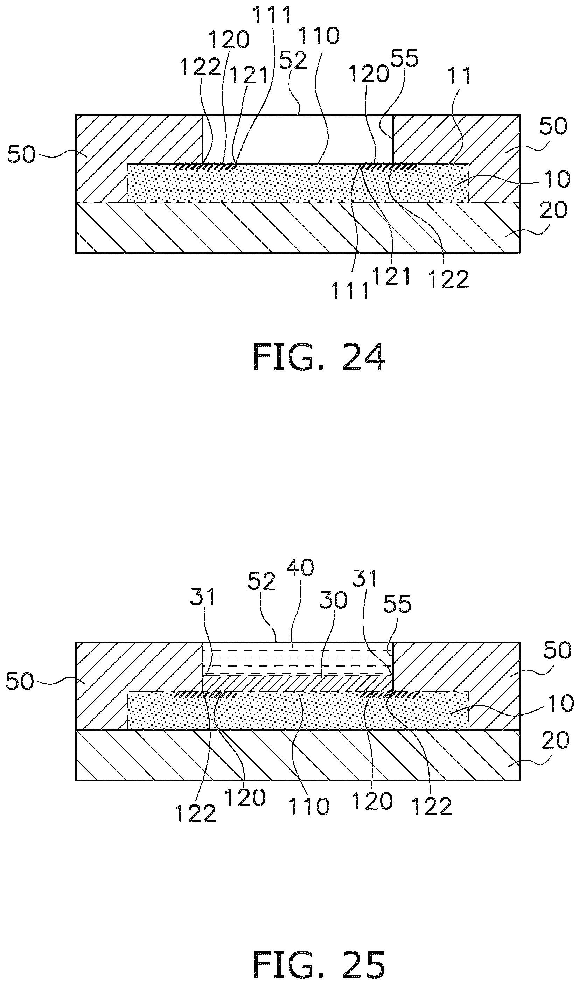

As discussed above, the outward spread of the first liquid composition P applied onto the first region 110 and the second region 120 of the electrode 10 is prevented by the third inner peripheral edge portion 131 of the third region 130. The electrode 10 according to the embodiment shown in Figs. 2, 7, and 8 is advantageous in that it is lower in cost than the electrode 10 according to the embodiment shown in Figs. 23 and 24 (discussed below) in which an insulating layer 50 is disposed to prevent the applied first liquid composition P from spreading outwardly.

In the electrode 10 according to the embodiment shown in Figs. 2, 7, and 8, the maximum width M (indicating the diameter in the depicted example) of the outer periphery of the second region 120 can be adjusted as appropriate to suit the desired dimensions of the reagent layer 30, and can be, for example, at least 300 $\mu$m and no more than 5000 $\mu$m, and preferably at least 900 $\mu$m and no more than 2000 $\mu$m. The width N in the radial direction of the second region 120 can also be adjusted as appropriate according to the dimensions of the reagent layer 30 to be obtained, and can be, for example, at least 0.5% and no more than 40%, and preferably at least 5% and no more than 30%, of the maximum width M of the outer periphery. The width N in the radial direction of the second region 120 can be, for example, at least 50 $\mu$m and no more than 500 $\mu$m, and preferably at least 90 $\mu$m and no more than 400 $\mu$m.

Sensor 1

The sensor 1 according to an embodiment of the present invention will be described with reference to the drawings.

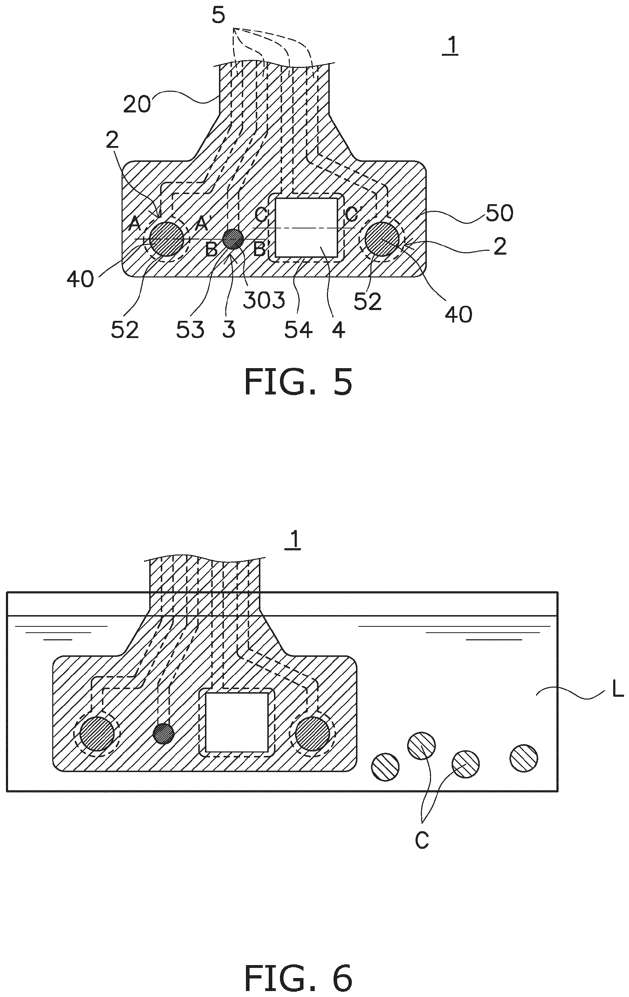

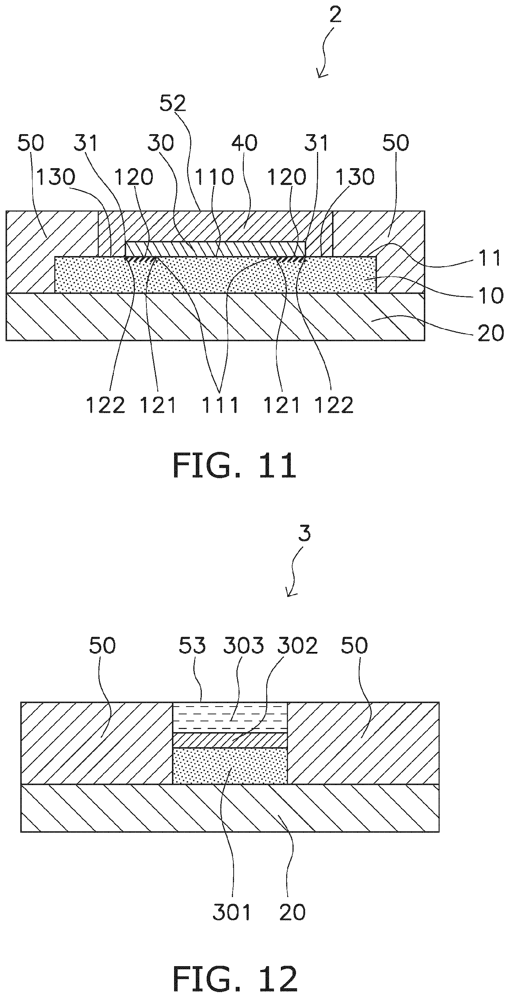

As shown in Fig. 5 and in Figs. 11, 12, and 13, which are cross-sectional views thereof, the sensor 1 of this embodiment comprises:

the electrode 10; and
the reagent layer 30 that is disposed in the first region 110 and the second region 120 of the electrode 10 and includes the outer peripheral edge portion 31 located on the second outer peripheral portion 122 of the second region 120, and a reagent that participates in an oxidation-reduction reaction.

In the sensor 1 of this embodiment, the electrode 10 and the reagent layer 30 constitute the working electrode 2. The electrode 10 is preferably disposed on the first surface 21 of the insulating substrate 20.

Fig. 11 is a cross-sectional view along the A-A' line of the portion of the sensor 1 shown in Fig. 5 that includes the working electrode 2. The first region 110 and the second region 120 are formed on the surface portion 11 of the electrode 10 located on the opposite side from the substrate 20, and the reagent layer 30 is disposed in the first region 110 and the second region 120. As shown in Fig. 11, the sensor 1 further includes a protective film 40 that covers the reagent layer 30. The protective film 40 prevents or suppresses leakage of the reagent contained in the reagent layer 30. The electrode 10 is connected to wiring 5 disposed on the first surface 21 of the substrate 20.

Specific examples of the materials used for the electrode 10, the substrate 20, the reagent layer 30, and the protective film 40 are as described in the "Materials" section.

The sensor 1 of this embodiment comprises two working electrodes 2, but in another embodiment (not shown), there may be just one working electrode, or there may be three or more. In a sensor 1 comprising two or more working electrodes 2, the reagent layers 30 of the two or more working electrodes 2 can contain reagents that participate in an oxidation-reduction reaction of different analytes. For example, some of the two or more working electrodes 2 may include a reagent layer 30 containing a reagent that participates in the oxidation-reduction reaction of lactate, and others may include a reagent layer 30 containing a reagent that participates in the oxidation-reduction reaction of glucose.

The electrode 10 in the sensor 1 of this embodiment includes an insulating layer 50, at least a portion of which is disposed on the surface portion 11. The insulating layer 50 has an opening 52 formed in the first region 110, the second region 120, and the third region 130 of the surface portion 11 of the electrode 10. In this embodiment, as shown in Fig. 11, the reagent layer 30 and the protective film 40 are disposed within the opening 52. The insulating layer 50 covers the surface portion 11 of the electrode 10, as well as other portions of the first surface 21 of the substrate 20.

The sensor 1 of this embodiment further comprises a reference electrode 3 and/or a counter electrode 4 disposed on the substrate 20, and preferably has both the reference electrode 3 and the counter electrode 4 as in the example shown in the drawings.

Fig. 12 is a cross-sectional view along the B-B' line of the portion of the sensor shown in Fig. 5 that includes the reference electrode 3. The reference electrode 3 includes a reference electrode conductive layer 301 that is disposed on the first surface 21 of the substrate 20, and a silver/silver chloride layer 302 that is disposed on the reference electrode conductive layer 301. The reference electrode 3 preferably further includes a reference electrode protective film 303 that is disposed on the silver/silver chloride layer 302 as shown in the drawing. Preferred embodiments of the material of the reference electrode conductive layer 301 and the reference electrode protective film 303 are as discussed in the "Materials" section. Wiring 5 is connected to the reference electrode conductive layer 301 of the reference electrode 3. The insulating layer 50 has a reference electrode opening 53 that is formed at a position overlapping the reference electrode 3 in plan view. The reference electrode conductive layer 301, the silver/silver chloride layer 302, and the reference electrode protective film 303 of the reference electrode 3 are disposed within the reference electrode opening 53 as shown in Fig. 12.

Fig. 13 is a cross-sectional view along the C-C' line of the portion of the sensor 1 shown in Fig. 5 that includes the counter electrode 4. The counter electrode 4 is a conductive layer disposed on the first surface 21 of the substrate 20. Preferred embodiments of the material of the conductive layer constituting the counter electrode 4 are as described in the "Materials" section. The wiring 5 is connected to the counter electrode 4. The insulating layer 50 has a counter electrode opening 54 formed at a position overlapping part of the upper surface 4a of the counter electrode 4 in plan view. As shown in Fig. 13, the counter electrode 4 is disposed within the counter electrode opening 54, and part of the upper surface 4a of the counter electrode 4 is exposed through the counter electrode opening 54.

As shown in Fig. 6, the sensor 1 is immersed in a liquid sample L and used to detect a specific analyte in the liquid sample L. Specific examples of the liquid sample and the analyte are as described in the "Materials" section. Fig. 6 shows a cell culture solution containing cells C as an example of the liquid sample L.

In the case where the reagent layer 30 of the working electrode 2 of the sensor 1 contains a reagent that oxidizes the analyte in the liquid sample L, electrons move from the analyte to the electrode 10 of the working electrode 2 under conditions in which a specific voltage is applied to the working electrode 2, the reference electrode 3, and the counter electrode 4 of the sensor 1. Similarly, in the case where the reagent layer 30 contains a reagent that reduces the analyte in the liquid sample L, electrons move from the electrode 10 of the working electrode 2 to the analyte. Since the amount of electron movement depends on the concentration of the analyte, the concentration, or change in concentration, of the analyte in the liquid sample L can be measured on the basis of the amount of current, or the change in the amount of current, flowing through the electrode 10 of the working electrode 2 of the sensor 1.

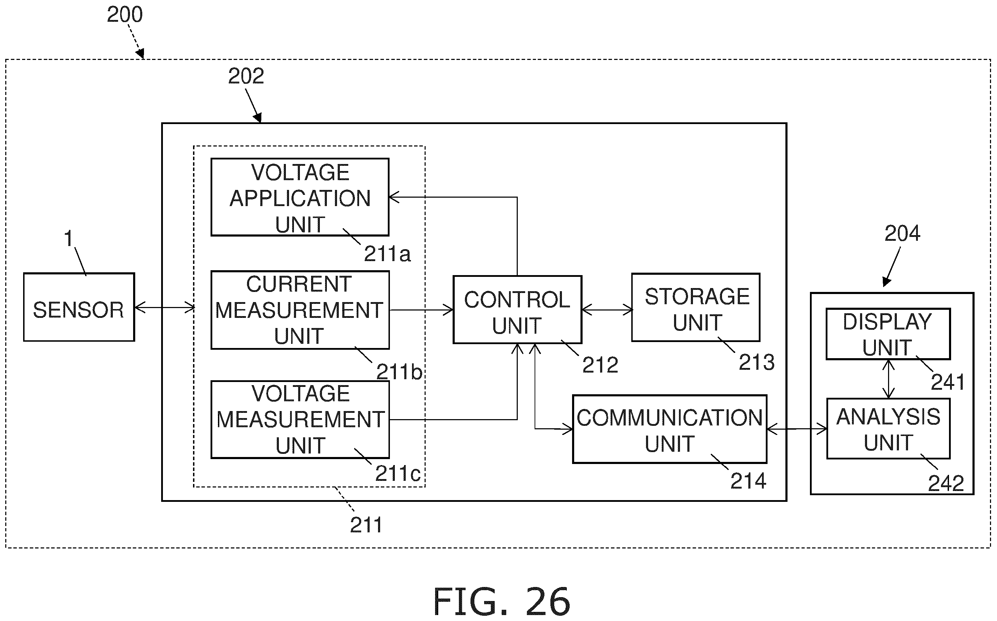

An example of an analysis device 200 for analyzing an analyte in a liquid sample, which comprises the sensor 1, will be described with reference to Fig. 26.

The analysis device 200 includes the sensor 1, an analyzer 202, and a controller 204. The working electrode 2, the reference electrode 3, and the counter electrode 4 of the sensor 1 are each connected to the analyzer 202 via the wiring 5. The analyzer 202 is able to communicate with the controller 204.

The analyzer 202 includes an electrochemical measurement unit 211, a control unit 212, a storage unit 213, and a communication unit 214.

The electrochemical measurement unit 211 is a potentiostat that measures the concentration of the analyte by applying a specific voltage to the working electrode 2, the reference electrode 3, and the counter electrode 4 of the sensor 1, and includes a voltage application unit 211a and a current measurement unit 211b, and preferably further includes a voltage measurement unit (counter electrode terminal voltage measurement unit) 211c.

The voltage application unit 211a applies a specific voltage to the working electrode 2, the reference electrode 3, and the counter electrode 4 of the sensor 1 in order to measure the concentration of the analyte contained in the liquid sample L.

The current measuring unit 211b senses the amount of current flowing between the working electrode 2 and the counter electrode 4 of the sensor 1, or a change in this amount, which is measured while a voltage is applied from the voltage application unit 211a to the working electrode 2, the reference electrode 3, and the counter electrode 4 of the sensor 1. As discussed above, the amount of current, or the change in the amount of current, sensed by the current measuring unit 211b serves as an index of the concentration, or the change in concentration, of the analyte in the liquid sample L.

The voltage measuring unit 211c measures the terminal voltage of the counter electrode 4 of the sensor 1.

The control unit 212 is connected to the voltage application unit 211a, the current measurement unit 211b, the voltage measurement unit 211c, the storage unit 213, and the communication unit 214. The control unit 212 controls the voltage application unit 211a so as to apply a specific voltage to the working electrode 2, the reference electrode 3, and the counter electrode 4 of the sensor 1, and controls the communication unit 214 so as to transmit the measurement results obtained by the current measurement unit 211b and the voltage measurement unit 211c to the controller 204.

The memory unit 213 is connected to the control unit 212 and stores, for example, the value of the applied voltage preset for each measurement object, the measurement values obtained by the current measurement unit 211b and the voltage measurement unit 211c, a calibration curve measured in advance, and other such data.

The communication unit 214 is controlled by the control unit 212 and transmits the measurement results obtained by the current measurement unit 211b and the voltage measurement unit 211c and other such data to the analysis unit 242 of the controller 204.

The controller 204 can communicate with the analyzer 202 via the communication unit 214, and includes a display unit 241 and an analysis unit 242.

The display unit 241 displays, for example, the concentration of the analyte in the liquid sample L based on the amount of current sensed by the current measurement unit 211b, as the result of the analysis by the analysis unit 242.

The analysis unit 242 is, for example, a PC (personal computer), and calculates the concentration of the analyte on the basis of the amount of current flowing between the working electrode 2 and the counter electrode 4, as measured by the current measurement unit 211b.

Method for Manufacturing Sensor 1

An example of a method for manufacturing the sensor 1 according to the embodiment shown in Fig. 5 will be described with reference to the drawings, and particularly Figs. 1 to 5.

First, as shown in Fig. 1, an electrode (working electrode conductive layer) 10, a reference electrode conductive layer 301, a counter electrode (counter electrode conductive layer) 4, and wiring 5 electrically connected to each of these are disposed on the first surface 21 of the insulating substrate 20.

Then, as shown in Fig. 2, the first region 110, the second region 120, and the third region 130 are formed on the surface portion 11 of the electrode 10 located on the opposite side from the substrate 20. Here, in the case where the entire untreated surface portion 11 of the electrode 10 has a first surface free energy, a part of the surface portion 11 serves as the first region 110, and a treatment for increasing the surface free energy as described above is selectively performed on the annular region surrounding the first region 110, allowing the formation of the first region 110 having the first surface free energy, the second region 120 having the second surface free energy that is higher than the first surface free energy, and the third region 130 having the third surface free energy that is the same as the first surface free energy.

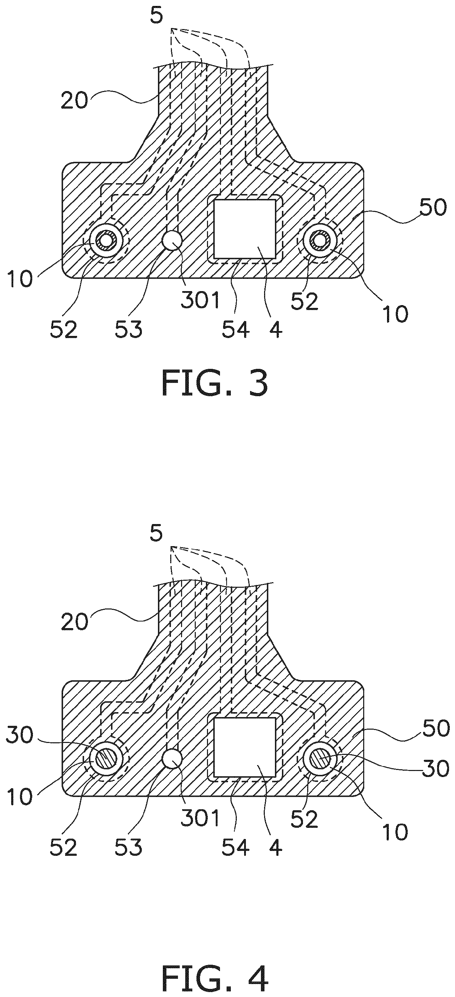

Then, as shown in Fig. 3, the insulating layer 50 is laminated on the first surface 21 of the substrate 20 on which the electrode 10, the reference electrode conductive layer 301, the counter electrode 4, and the wiring 5 are disposed. The insulating layer 50 has the opening 52 formed in the first region 110, the second region 120, and the third region 130 of the surface portion 11 of the electrode 10, the reference electrode opening 53 formed in the reference electrode conductive layer 301, and the counter electrode opening 54 formed in the counter electrode 4.

Then, as shown in Figs. 4 and 10, the outer peripheral edge portion 31 located on the second outer peripheral edge portion 122 of the second region 120, and the reagent layer 30 containing a reagent that participates in an oxidation-reduction reaction are formed in the first region 110 and the second region 120 of the electrode 10 inside the opening 52 of the insulating layer 50. As described above with reference to Figs. 9 and 10, the reagent layer 30 can be formed by coating the first region 110 and the second region 120 of the electrode 10 with a first liquid composition P containing the reagent in a first solvent, and then drying the first liquid composition P. Preferred embodiments of the method for forming the reagent layer 30, the first solvent, the first liquid composition P, and the electrode 10 on which the reagent layer 30 is formed are as discussed above. The reagent layer 30 formed on the electrode 10 by this method includes a large amount of the reagent. Accordingly, the sensor 1 manufactured by the method according to this embodiment is suited to a method in which the sensor 1 is immersed in a liquid sample L for an extended period of time to continuously detect an analyte.

Then, as shown in Figs. 5 and 11, a protective film 40 is formed covering the reagent layer 30. The protective film 40 can be formed by coating the surface portion 11 of the electrode 10 with a second liquid composition containing protective film components in a second solvent to so as to cover the reagent layer 30, and then drying the second liquid composition. In the example shown in Figs. 5 and 11, the protective film 40 can be formed by coating the inside of the opening 52 of the insulating layer 50, including the reagent layer 30 formed on the surface portion 11 of the electrode 10, with the second liquid composition, and drying the second liquid composition. An example of the second solvent is alcohol. Specific examples of the second solvent, the protective film components, and polymer compounds and crosslinking agents constituting the protective film are as discussed in the "Materials" section.

Furthermore, as shown in Figs. 5 and 12, a silver/silver chloride layer 302 and a reference electrode protective film 303 are laminated on the reference electrode conductive layer 301 in the reference electrode opening 53 of the insulating layer 50 to complete the reference electrode 3. The reference electrode protective film 303 can be formed by the same method as the protective film 40 of the working electrode 2.

Embodiment of Electrode 10 shown in Fig. 14

Another embodiment of the electrode 10 will be described with reference to Fig. 14. Fig. 14 is a detail view of the region of the electrode 10 according to this embodiment corresponding to the region X in the electrode 10 shown in Fig. 2.

The electrode 10 according to this embodiment shown in Fig. 14 includes the surface portion 11, the first region 110 formed on the surface portion 11 and having the following characteristics, and the second region 120 formed on the surface portion 11 and having a second surface free energy higher than the first surface free energy. The electrode 10 according to this embodiment further includes a third region 130 that is formed on the surface portion 11 and has a third surface free energy lower than the second surface free energy.

In the electrode 10 according to this embodiment, the first region 110 comprises:
a first outer peripheral edge portion 111, and further includes:

a center region 112 disposed in the interior of the first region 110 that includes an outer peripheral edge portion 113 located more to the inside than the first outer peripheral edge portion 111 of the first region 110, and has a surface free energy higher than the first surface free energy, and
a surrounding region 115 that surrounds the center region 112, includes the first outer peripheral edge 111 and an inner peripheral edge portion 114 in contact with the outer peripheral edge portion 113 of the center region 112, and has a first surface free energy.

In the electrode 10 according to the present embodiment shown in Fig. 14, the surrounding region 115 is the region of the first region 110 other than the center region 112, surrounds the center region 112, and includes the inner peripheral edge portion 114 in contact with the entire outer peripheral edge portion 113 of the center region 112, and the entire first outer peripheral edge 111. The center region 112 is preferably disposed at a position including the geometric center of the first region 110, and more preferably, the geometric center of the center region 112 coincides with the geometric center of the first region 110.

In the electrode 10 according to this embodiment shown in Fig. 14, the surface free energy of the center region 112 is greater than the first surface free energy. The specific difference between the surface free energy of the center region 112 and the first surface free energy can be selected from the same range as the difference discussed in relation to the difference between the second surface free energy and the first surface free energy. In particular, the polar component of the surface free energy of the center region 112 is preferably greater than the polar component of the first surface free energy, and the specific value of the difference can be selected from the same range as the value discussed in relation to the difference between the polar component of the second surface free energy and the polar component of the first surface free energy. The value of the surface free energy of the center region 112, the value of the polar component of the surface free energy of the center region 112, and the ratio of the dispersive component to the polar component ($\gamma sd/\gamma sp$) of the surface free energy of the center region 112 can be selected from the ranges discussed in relation to the second surface free energy, and are particularly preferably the same as those discussed in relation to the second surface free energy.

The electrode 10 according to this embodiment shown in Fig. 14 has the following effect.

Unlike in Fig. 14, in a case where the first liquid composition P is applied as shown in Fig. 9 in the first region 110 and the second region 120 of the electrode 10 according to the embodiment shown in Fig. 7, in which the entire first region 110 has a first surface free energy, and the surrounding second region 120 has a second surface free energy, a nozzle (not shown) is usually brought close to the first region 110, the first liquid composition P is applied in drops from the nozzle, and then the nozzle is moved away from the electrode 10. At this point, with the electrode 10 according to the embodiment shown in Fig. 7, the entire first region 110 has low wettability by the first liquid composition P, so when a drop of the first liquid composition P fall, the height of the droplet of the first liquid composition P formed on the first region 110 increases to the point that there is no separation from the distal end of the nozzle, and when the nozzle is moved away from the electrode 10, some of the first liquid composition P clinging to the nozzle may also move away from the electrode 10.

With the electrode 10 according to this embodiment shown in Fig. 14, the first region 110 includes a center region 112 having a surface free energy greater than the first surface free energy, and a surrounding region 115 having the first surface free energy and surrounding the center region 112. The center region 112 has higher wettability with respect to the first liquid composition P than the surrounding region 115 of the first region 110. Therefore, when the nozzle is brought close to the center region 112 of the first region 110 and a drop of the first liquid composition P falls from the nozzle, the height of the droplet of the first liquid composition P in the center region 112 is low enough that there is separation from the distal end of the nozzle, and when the nozzle moves away from the electrode 10, the first liquid composition P is unlikely to move away from the electrode 10 together with the nozzle. Therefore, with the electrode 10 according to this embodiment shown in Fig. 14, it is easier to apply the intended amount of the first liquid composition P in the first region 110 and the second region 120.

In the electrode 10 according to this embodiment shown in Fig. 14, the maximum width M (the diameter in the depicted example) of the outer periphery of the second region 120 and the width N in the radial direction of the second region 120 can be the same values as M and N in the electrode 10 according to the embodiment shown in Figs. 2, 7, and 8. The maximum width W (the diameter in the depicted example) of the center region 112 can be, for example, at least 0.5% and no more than 30%, and preferably at least 5% and no more than 25%, of the maximum width M of the outer periphery of the second region 120. The maximum width W of the center region 112 can be, for example, at least 50 $\mu$m and no more than 500 $\mu$m, and preferably at least 90 $\mu$m and no more than 300 $\mu$m.

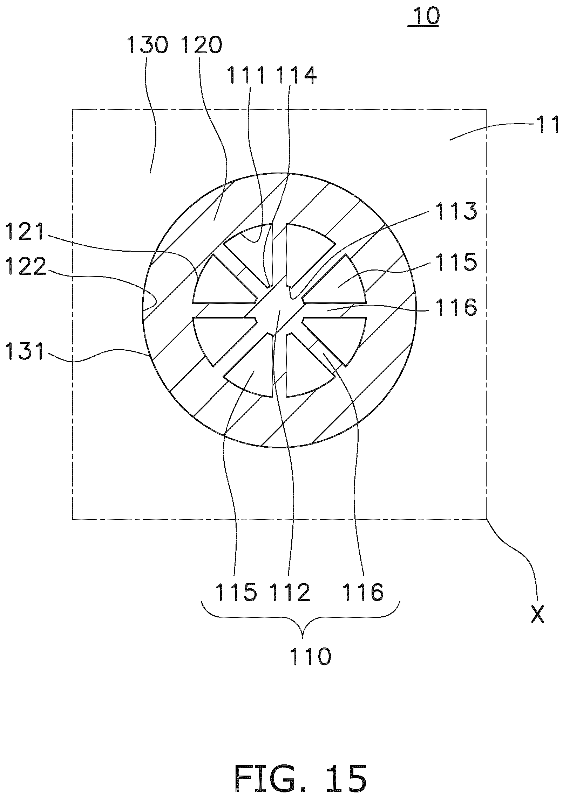

Embodiment of Electrode 10 shown in Fig. 15

Another embodiment of the electrode 10 will be described with reference to Fig. 15.

This embodiment shown in Fig. 15 is a modification example of the embodiment shown in Fig. 14, and features common to the embodiment shown in fig. 14 will not be described repeatedly.

In the electrode 10 according to this embodiment, the first region 110 comprises:
a first outer peripheral edge portion 111, and further includes:

a center region 112 disposed inside the first region 110, including an outer peripheral edge portion 113 located more to the inside than the first outer peripheral edge portion 111 of the first region 110, and having a surface free energy greater than the first surface free energy,
a surrounding region 115 surrounding the center region 112, including the first outer peripheral edge 111 and an inner peripheral edge portion 114 in contact with the outer peripheral edge portion 113 of the center region 112, and having the first surface free energy, and
a connection region 116 extending through the surrounding region 115 from part of the outer peripheral edge portion 113 of the center region 112 to part of the second inner peripheral edge portion 121 of the second region 120 facing the center region 112 via the surrounding region 115, and having a surface free energy greater than the first surface free energy.

The connection region 116 extends in the radial direction of the first region 110. Preferably, a plurality of (such as 3 to 10) connection regions 116 are included as shown in the drawings. The plurality of connection regions 116 are preferably disposed at equal intervals in the circumferential direction of the first outer peripheral edge portion 111.

In the electrode 10 according to this embodiment shown in fig. 15, the surrounding region 115 is divided into a plurality of segments by the plurality of connection regions 116, each of which has a first surface free energy. With the electrode 10 according to this embodiment shown in Fig. 15, the first outer peripheral edge portion 111 and the inner peripheral edge portion 114 are also divided into a plurality of segments by the plurality of connection regions 116.

The surface free energy of the center region 112 and the surface free energy of the connection region 116 are each greater than the first surface free energy. The surface free energy of the center region 112 and the surface free energy of the connection region 116 may be the same or different, but are preferably the same. The surface free energy of the center region 112 and the surface free energy of the connection region 116 may be the same as or different from the second surface free energy of the second region 120, but preferably both are the same as the second surface free energy of the second region 120. The preferred range of the surface free energy of the center region 112 is as discussed in relation to the embodiment shown in Fig. 14. The specific difference between the surface free energy of the connection region 116 and the first surface free energy can be selected from the same range as the difference discussed in relation to the difference between the second surface free energy and the first surface free energy. In particular, it is preferable for the polar component of the surface free energy of the connection region 116 to be greater than the polar component of the first surface free energy, and the specific value of this difference can be selected from the same range as the value discussed in relation to the difference between the polar component of the second surface free energy and the polar component of the first surface free energy. The value of the surface free energy of the connection region 116, the value of the polar component of the surface free energy of the connection region 116, and the ratio of the dispersive component to the polar component ($\gamma$sd/$\gamma$sp) of the surface free energy of the connection region 116 can be selected from the range discussed in relation to the second surface free energy, and are particularly preferably the same as those discussed in relation to the second surface free energy.

The electrode 10 according to this embodiment shown in Fig. 15 exhibits the following effect. Compared to the surrounding region 115 having the first surface free energy of the first region 110, the center region 112, the connection region 116, and the second region 120 having a higher surface free energy have higher wettability by the first liquid composition P. Accordingly, the first liquid composition P dropped from the nozzle into the central region 112 of the first region 110 readily spreads out from the center region 112 to the second region 120 through the connection region 116. Therefore, with the electrode 10 according to this embodiment, after the nozzle is brought close to the central region 112 of the first region 110 and the first liquid composition P is dropped from the nozzle, the nozzle is moved away from the electrode 10, at which point it is even less likely that the first liquid composition P will cling to the nozzle and move away from the electrode 10 than with the electrode 10 according to the embodiment shown in Fig. 14.

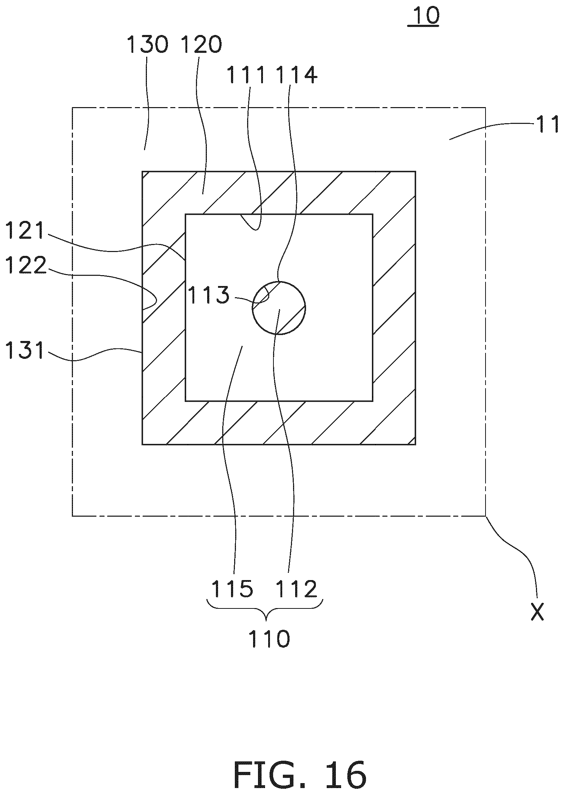

Embodiment of Electrode 10 shown in Fig. 16

In the electrode 10 according to the embodiment shown in Figs. 7, 14, and 15, the first outer peripheral edge portion 111 of the first region 110, the second inner peripheral edge portion 121 of the second region 120, the second outer peripheral edge portion 122 of the second region 120, and the third inner peripheral edge portion 131 of the third region 130 each have a circular shape in plan view. In the electrode 10 according to the embodiment shown in Figs. 14 and 15, the outer peripheral edge portion 113 of the center region 112 and the inner peripheral edge portion 114 of the surrounding region 115 of the first region 110 each have a circular shape in plan view. However, a circular shape is not the only option, and other shapes such as polygons (triangles, squares, hexagons, etc.) may be used instead.

For example, the electrode 10 according to the embodiment shown in Fig. 16 is an example in which the first outer peripheral edge portion 111 of the first region 110, the second inner peripheral edge portion 121 of the second region 120, the second outer peripheral edge portion 122 of the second region 120, and the third inner peripheral edge portion 131 of the third region 130 each have a rectangular plan-view shape. In the electrode 10 according to the embodiment shown in Fig. 16, the plan-view shapes of the center region 112 and the outer peripheral edge portion 113 of the inner peripheral edge portion 114 of the surrounding region 115 of the first region 110 are each circular, but this is not the only option, and other shapes such as polygons may be used instead. Other features of the electrode 10 according to the embodiment shown in Fig. 16 are the same as those of the electrode 10 according to the embodiment shown in Fig. 14, and will not be described repeatedly.

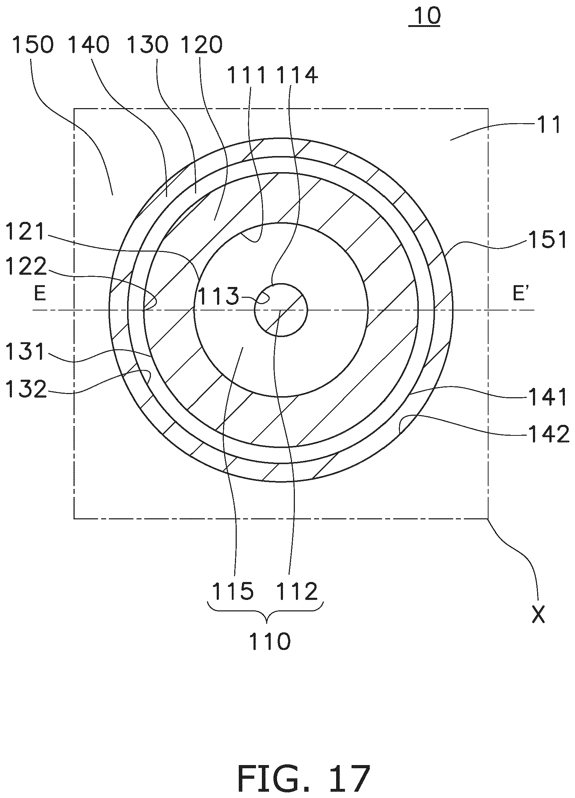

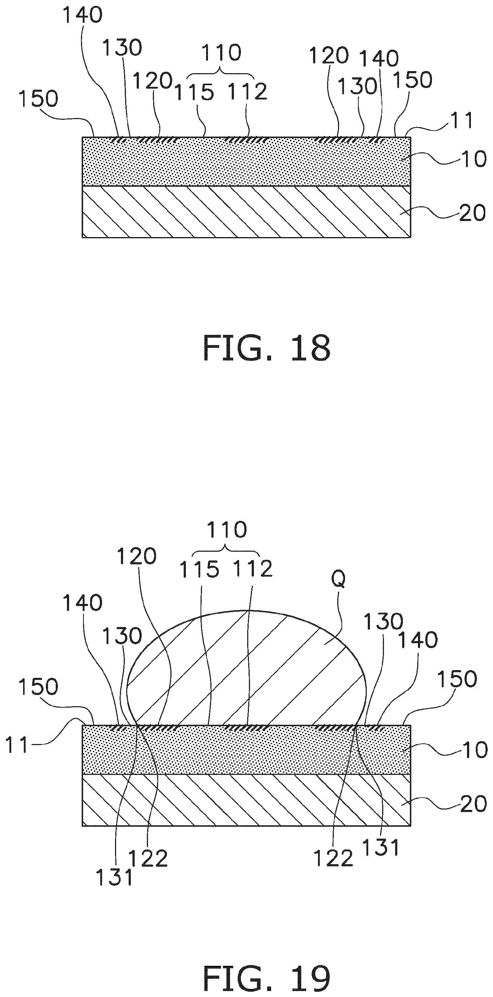

Embodiment of Electrode 10 shown in Figs. 17 and 18

A further embodiment of the electrode 10 will be described with reference to Figs. 17 and 18. Fig. 18 is a cross-sectional view along the E-E' line in Fig. 17. Fig. 17 is a detail view of the region of the electrode 10 according to this embodiment corresponding to the region X in the electrode 10 shown in Fig. 2.

The electrode 10 according to this embodiment shown in Figs. 17 and 18 comprises:

a surface portion 11 located on the opposite side from the insulating substrate 20 when the electrode is disposed on the substrate 20;
a first region 110 that is formed on the surface portion 11, includes a first outer peripheral edge portion 111, and at least a portion of which has a first surface free energy;
a second region 120 that is formed on the surface portion 11, surrounds the first region 110, includes a second inner peripheral edge portion 121 in contact with the first outer peripheral edge portion 111 of the first region 110, and a second outer peripheral edge portion 122 located more to the outside than the second inner peripheral edge portion 121, and has a second surface free energy that is greater than the first surface free energy;
a third region 130 that is formed on the surface portion 11, surrounds the second region 120, includes a third inner peripheral edge portion 131 in contact with the second outer peripheral edge portion 122 of the second region 120, and a third outer peripheral edge portion 132 located more to the outside than the third inner peripheral edge 131, and has a third surface free energy that is lower than the second surface free energy; and
a fourth region 140 that is formed on the surface portion 11, surrounds the third region 130, includes a fourth inner peripheral edge portion 141 in contact with the third outer peripheral edge portion 132 of the third region 130, and a fourth outer peripheral edge portion 142 located more to the outside than the fourth inner peripheral edge 141, and has a fourth surface free energy that is higher than the third surface free energy.

The features of the first region 110 and the second region 120 in this embodiment shown in Figs. 17 and 18 are the same as those described for the electrode 10 according to the other embodiments, and therefore will not be described repeatedly. The first region 110 in this embodiment shown in Figs. 17 and 18 includes a center region 112 having a surface free energy greater than the first surface free energy, and a surrounding region 115 having the first surface free energy, just as in the embodiment shown in Fig. 14.

The third surface free energy of the third region 130 may be lower than the second surface free energy of the second region 120, and may be the same as or different from the first surface free energy of the surrounding region 115 of the first region 110. Preferably, the third surface free energy of the third region 130 is the same as the first surface free energy of the surrounding region 115 of the first region 110. The difference between the third surface free energy of the third region 130 and the second surface free energy and the preferred range of the third surface free energy are as discussed in the "Surface Free Energy" section.

The fourth surface free energy of the fourth region 140 is higher than the third surface free energy of the third region 130, and may be the same as or different from the second surface free energy of the second region 120. The difference between the fourth surface free energy and the third surface free energy, and specific examples of the value of the fourth surface free energy are as discussed in the "Surface Free Energy" section.

Using the electrode 10 having the surface portion 11, the first region 110, the second region 120, the third region 130, and the fourth region 140 makes it possible to accurately form the reagent layer 30 and the protective film 40. This mechanism will be described with reference to Figs. 19 to 22.

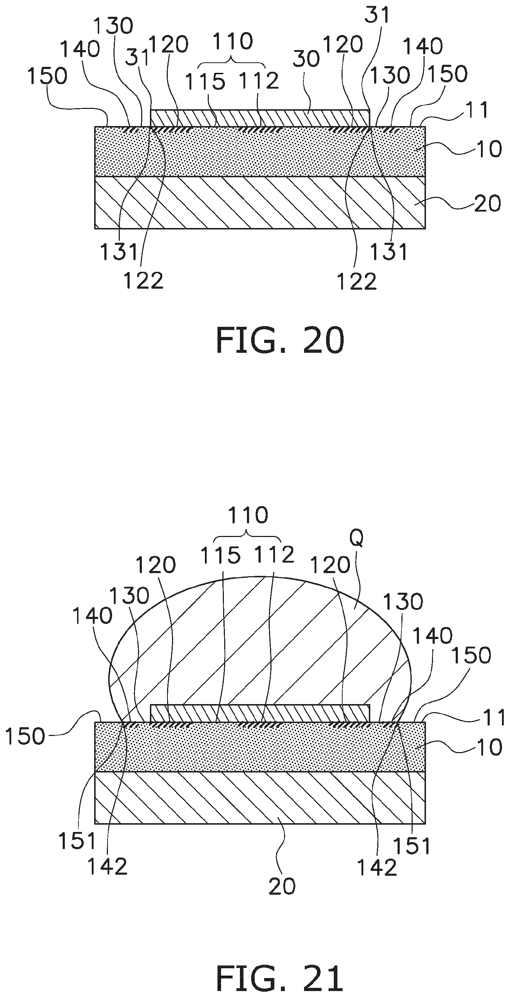

As shown in Fig. 19, the first region 110 and the second region 120 of the electrode 10 are coated with the first liquid composition P containing a reagent that participates in an oxidation-reduction reaction in a first solvent, and this coating is then dried to form the reagent layer 30 including an outer peripheral edge portion 31 located on the second outer peripheral edge portion 122 of the second region 120, as shown in Fig. 20. This process is the same as that described with reference to Figs. 9 and 10, in which the reagent layer 30 is formed on the electrode 10 according to the embodiment shown in Figs. 7 and 8, and therefore will not be described repeatedly. With the electrode 10 of this embodiment shown in Figs. 17 and 18, it is possible to accurately form a reagent layer 30 having the designed dimensions in the first region 110 and the second region 120 of the surface portion 11. The contact surface area between the reagent layer 30 and the electrode 10 is determined by the first region 110 and the second region 120 of the electrode 10.

The electrode 10 according to this embodiment shown in Figs. 17 and 18 has a third region 130 with a third surface free energy, which is less wettable by the first liquid composition P than the second region 120 having the second surface free energy. Therefore, as shown in Fig. 19, the first liquid composition P applied to the first region 110 and the second region 120 is prevented from spreading outwardly by the third inner peripheral edge portion 131 of the third region 130, and the outer periphery is defined by the second outer peripheral edge portion 122 of the second region 120.

Then, as shown in Fig. 21, the first region 110, the second region 120, the third region 130, and the fourth region 140 of the electrode 10 are coated with a second liquid composition Q containing a protective film component in a second solvent, so as to cover the reagent layer 30. Examples of the second solvent and the protective film component of the second liquid composition Q have been given above. The applied second liquid composition Q forms a droplet whose outer periphery is defined by the fourth outer peripheral edge portion 142 of the fourth region 140.

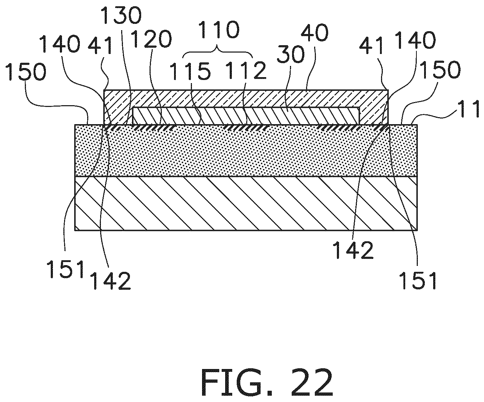

Next, the applied second liquid composition Q is dried to form a protective film 40 as shown in Fig. 22. The protective film 40 thus formed is disposed on the first region 110, the second region 120, the third region 130, and the fourth region 140 of the electrode 10 so as to cover the reagent layer 30, and includes an outer peripheral edge portion 41 located on the fourth outer peripheral edge portion 142 of the fourth region 140. The surface area of the protective film 40 is determined by the fourth outer peripheral edge portion 142 of the fourth region 140. Thus, the outer shape and surface area of the protective film 40 can be adjusted by adjusting the outer shapes and surface areas of the first region 110, the second region 120, the third region 130, and the fourth region 140. Meanwhile, the thickness of the protective film 40 can be adjusted by adjusting the concentration of the protective film components in the second liquid composition Q and the amount in which the second liquid composition Q is applied. With the electrode 10 of this embodiment, it is possible to accurately form a protective film of the designed dimensions in the first region 110, the second region 120, the third region 130, and the fourth region 140.

The electrode 10 according to this embodiment shown in Figs. 17 and 18 further includes a fifth region 150 that is formed on the surface portion 11, surrounds the fourth region 140, includes a fifth inner peripheral edge portion 151 in contact with the fourth outer peripheral edge portion 142 of the fourth region 140, and has a fifth surface free energy lower than the fourth surface free energy. The second liquid composition Q has a lower wettability with respect to the fifth region 150 having the fifth surface free energy than the fourth region 140 having the fourth surface free energy. Therefore, the second liquid composition Q applied to the first region 110, the second region 120, the third region 130, and the fourth region 140 of the electrode 10 so as to cover the reagent layer 30 is prevented from spreading outwardly by the fifth inner peripheral edge portion 151 of the fifth region 150, so the above-mentioned effect that the outer periphery of the droplet is defined by the fourth outer peripheral edge portion 142 of the fourth region 140 is more easily realized. The difference between the fourth surface free energy and the fifth surface free energy, and specific examples of the value of the fifth surface free energy are as discussed in the "Surface Free Energy" section. In an embodiment in which the surface portion 11 of the electrode 10 having a uniform surface free energy is partially subjected to a treatment to increase the surface free energy, thereby forming the second region 120 having the second surface free energy and the fourth region 140 having the fourth surface free energy, the first region 110, the third region 130, and the fifth region 150 all have the same surface free energy.

Embodiment of Electrode 10 shown in Figs. 23 and 24

With the electrode 10 according to the embodiment shown in Figs. 7, 8, 14, 15, 16, 17, and 18, the second outer peripheral edge portion 122 of the second region 120 is defined by the third inner peripheral edge portion 131 of the third region 130. However, this embodiment is not the only option, and the second outer peripheral edge portion 122 of the second region 120 may be defined by the insulating layer 50, as in the electrode 10 according to the embodiment shown in Figs. 23 and 24. Fig. 23 is a detail view of the region of the electrode 10 according to this embodiment corresponding to the region X in the electrode 10 shown in Fig. 2. Fig. 24 is a cross-sectional view along the F-F' line in Fig. 23.

The electrode 10 according to this embodiment shown in Figs. 23 and 24 includes the surface portion 11, the first region 110, the second region 120, and the insulating layer 50, at least a part of which is disposed on the surface portion 11. The insulating layer 50 has the opening 52 formed in the first region 110 and the second region 120, passing through in the thickness direction. The opening 52 of the insulating layer 50 includes an inner peripheral edge portion 55 that defines the second outer peripheral edge portion 122 of the second region 120 in plan view. The electrode 10 according to this embodiment shown in Figs. 23 and 24 and having this configuration exhibits the following effect. In the case where a recess surrounded by the opening 52 in the insulating layer 50, the bottom of which is the first region 110 and the second region 120, is coated with a first liquid composition containing a reagent participating in an oxidation-reduction reaction in a first solvent, the inner peripheral edge 55 of the insulating layer 50 prevents the first liquid composition from spreading outwardly, and only the first region 110 and the second region 120 are coated. The reagent layer 30 formed by drying the applied first liquid composition includes an outer peripheral edge portion 31 located on the second outer peripheral edge portion 122 of the second region 120 defined by the inner peripheral edge portion 55 of the insulating layer 50, and is disposed only in the first region 110 and the second region 120 (see Fig. 25). Therefore, with the electrode 10 according to this embodiment shown in Figs. 23 and 24, it is possible to form the reagent layer 30 of the designed dimensions in the first region 110 and the second region 120 even more accurately. Specific examples of the material constituting the insulating layer 50 are as discussed in the "Materials" section above. The insulating layer 50 preferably has a water-repellent surface, and more preferably a water-repellent surface containing a fluororesin.

The electrode 10 according to this embodiment shown in Figs. 23 and 24 can be manufactured by stacking the insulating layer 50 including the opening 52 whose inner peripheral edge portion 55 is located more to the outside than the second inner peripheral edge portion 121 of the second region 120 and in the second region 120, on the surface portion 11 of the electrode 10 having a surface portion 11 on which the first region 110 and the second region 120 have been formed. Here, the second outer peripheral edge portion 122 of the second region 120 in the opening 52 is defined by the inner peripheral edge portion 55 of the opening 52 of the insulating layer 50. The features of other components (such as the first region 110 and the second region 120) in the electrode 10 according to this embodiment shown in Figs. 23 and 24 are the same as those of the various components in the electrode 10 according to the embodiment shown in Figs. 7, 8, 14, 15, 16, 17, and 18.

The reagent layer 30 can be disposed in the first region 110 and the second region 120 of the electrode 10 according to this embodiment shown in Figs. 23 and 24, and the protective film 40 can be further disposed thereon (see Fig. 25). The protective film 40 can be formed by coating a recess surrounded by the opening 52 of the insulating layer 50 in which the reagent layer 30 is disposed, the bottom of which is the first region 110 and the second region 120 of the electrode 10, with a second liquid composition containing protective film components in a second solvent, and then drying the second liquid composition. Here again, the inner peripheral edge portion 55 of the insulating layer 50 prevents the applied second liquid composition from spreading outwardly, so the protective film 40 after drying will be disposed so as to cover the reagent layer 30 only in the first region 110 and the second region 120.

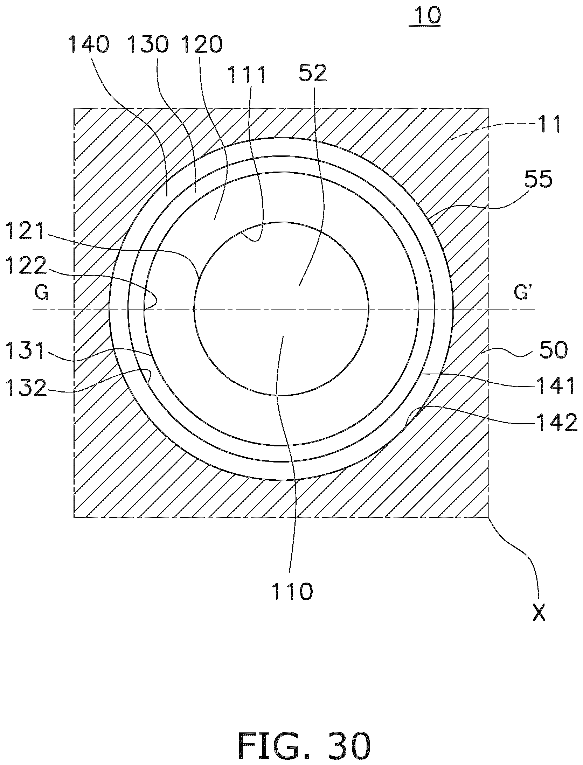

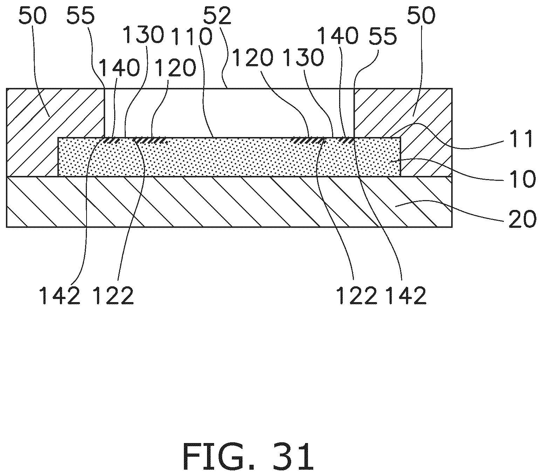

Embodiment of Electrode 10 shown in Figs. 30 and 31

Yet another embodiment of the electrode 10 will be described with reference to Figs. 30 and 31. Fig. 30 is a detail view of the region of the electrode 10 according to this embodiment corresponding to the region X in the electrode 10 shown in Fig. 2. Fig. 31 is a cross-sectional view along the G-G' line in the drawing. This embodiment is a modification example of the embodiment of the electrode 10 shown in Figs. 17 and 18.

The electrode 10 according to this embodiment shown in Figs. 30 and 31 includes:

a surface portion 11 that is located on the opposite side from the insulating substrate 20 when the electrode is disposed on the substrate 20;

a first region 110 that is formed on the surface portion 11, includes a first outer peripheral edge portion 111, and has a first surface free energy;

a second region 120 that is formed on the surface portion 11, surrounds the first region 110, includes a second inner peripheral portion 121 in contact with the first outer peripheral portion 111 of the first region 110, and a second outer peripheral portion 122 positioned more to the outside than the second inner peripheral portion 121, and has a second surface free energy that is greater than the first surface free energy;

a third region 130 that is formed on the surface portion 11, surrounds the second region 120, includes a third inner peripheral edge portion 131 in contact with a second outer peripheral edge portion 122 of the second region 120, and a third outer peripheral edge portion 132 positioned more to the outside than the third inner peripheral edge portion 131, and has a third surface free energy that is less than the second surface free energy;

a fourth region 140 that is formed on the surface portion 11, surrounds the third region 130, includes a fourth inner peripheral edge portion 141 in contact with the third outer peripheral edge portion 132 of the third region 130, and a fourth outer peripheral edge portion 142 positioned more to the outside than the fourth inner peripheral edge portion 141, and has a fourth surface free energy that is greater than the third surface free energy; and

an insulating layer 50 at least part of which is formed on the surface portion 11, and that is formed in the first region 110, the second region 120, the third region 130, and the fourth region 140, and includes an opening 52 passing through in the thickness direction and having an inner peripheral edge portion 55 in contact with the fourth outer peripheral edge portion 142 of the fourth region 140 in plan view from a direction perpendicular to the surface portion 11.

The features of the surface portion 11, the first region 110, the second region 120, the third region 130, the fourth region 140, and the insulating layer 50 in this embodiment shown in Figs. 30 and 31 other than those described below are the same as those described for the electrode 10 in other embodiments, and will not be described repeatedly. The entire first region 110 in this embodiment shown in Figs. 30 and 31 has the first surface free energy, just as in the embodiment shown in Fig. 7.

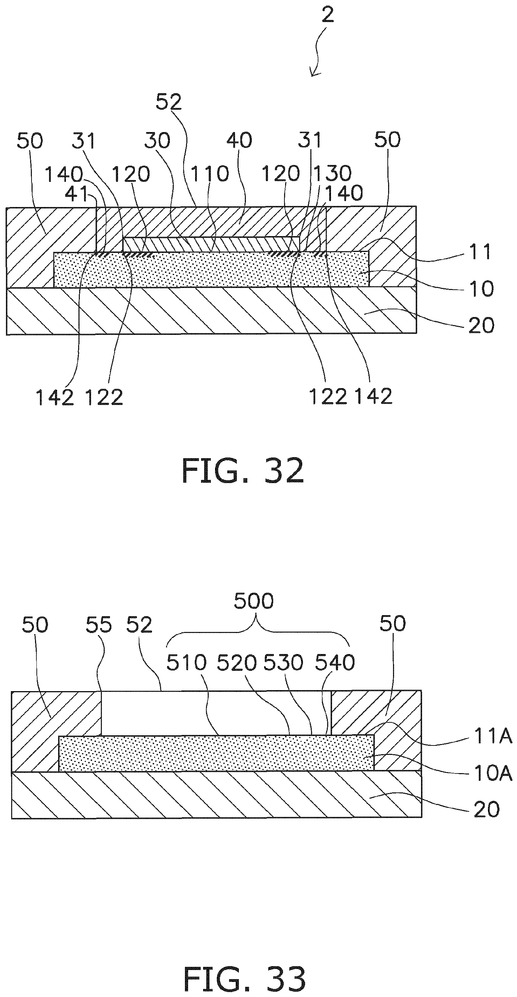

Fig. 32 is a cross-sectional view of the sensor 1 equipped with a working electrode 2 comprising the electrode 10 according to this embodiment shown in Figs. 30 and 31. The working electrode 2 (sensor 1) shown in Fig. 32 comprises a substrate 20, the electrode 10 according to this embodiment shown in figs. 30 and 31 disposed on the substrate 20, a reagent layer 30 disposed in the first region 110 and the second region 120 inside the opening 52 of the insulating layer 50, and a protective film 40 disposed in the first region 110, the second region 120, the third region 130, and the fourth region 140 inside the opening 52 of the insulating layer 50 so as to cover the reagent layer 30.

In the working electrode 2 (sensor 1) shown in Fig. 32, the reagent layer 30 includes an outer peripheral edge portion 31 located on the second outer peripheral portion 122 of the second region 120, and a reagent that participates in an oxidation-reduction reaction. The reagent layer 30 can be formed by the method described with reference to figs. 9 and 19.

In the working electrode 2 (sensor 1) shown in Fig. 32, the protective film 40 includes an outer peripheral edge portion 41 that is located on the fourth outer peripheral edge portion 142 of the fourth region 140 and is defined by the inner peripheral edge portion 55 of the insulating layer 50. As described with reference to Figs. 11 and 21, the protective film 40 is formed by coating the first region 110, the second region 120, the third region 130, and the fourth region 140 in the opening 52 of the insulating layer 50 with a second liquid composition containing protective film components in a second solvent so as to cover the reagent layer 30, and then drying the second liquid composition. Here, using the electrode 10 shown in Figs. 30 and 31 is advantageous in the following respects.

Since the inner peripheral edge portion 55 of the insulating layer 50 prevents the applied second liquid composition from spreading outwardly, the outer peripheral edge portion 41 of the dried protective film 40 can be located on the fourth outer peripheral edge portion 142 of the fourth region 140.

On the other hand, in the case where the insulating layer 50 having the opening 52 is formed on the surface portion 11 of the electrode 10 by a printing method such as screen printing, the inner peripheral edge portion 55 of the opening 52 may have fine recesses and protrusions in plan view from a direction perpendicular to the surface portion 11 due to limitations on machining accuracy. When the electrode 10 having such an insulating layer 50 is such that the outer peripheral edge portion of the bottom surface of the recess formed by the opening 52 of the insulating layer 50 and the surface portion 11 of the electrode 10 is the third region 130 having a third surface free energy with low wettability by the second liquid composition as shown in Fig. 11, the following problem may be encountered. Specifically, the applied second liquid composition cannot adhere to the inner peripheral edge portion 55 of the opening 52 of the insulating layer 50 and does not wet and spread out over the entire opening 52 of the insulating layer 50, so the surface area of the protective film 40 formed after drying ends up being smaller than designed, which means that the protective film 40 is more apt to peel off. Another problem that can occur is that the outer peripheral edge 41 of the protective film 40 after drying does not sufficiently adhere to the third region 130 of the surface portion 11 of the electrode 10 within the opening 52 of the insulating layer 50, so the protective film 40 is more likely to peel off. With the electrode 10 shown in Figs. 30 and 31, the outer peripheral edge portion of the bottom surface of the recess formed by the opening 52 of the insulating layer 50 and the surface portion 11 of the electrode 10 is a fourth region 140 having a fourth surface free energy with high wettability by the second liquid composition. Therefore, even in the case where the inner peripheral edge portion 55 of the opening 52 of the insulating layer 50 has fine recesses and protrusions, the applied second liquid composition can wet and spread out to the fourth outer peripheral edge portion 142 of the fourth region 140, and can adhere to the inner peripheral edge portion 55 of the opening 52 of the insulating layer 50 that is in contact with the fourth outer peripheral edge portion 142 in plan view. As a result, the dried protective film 40 can be formed in the designed surface area over the entire opening 52 of the insulating layer 50, and the outer peripheral edge portion 41 of the protective film 40 can adhere to the fourth outer peripheral edge portion 142 of the fourth region 140, which is preferable because peeling is less likely to occur.

Next, the method for manufacturing the electrode 10 according to this embodiment shown in figs. 30 and 31 will be described with reference to Figs. 33 to 36.

The method for manufacturing the electrode 10 according to this embodiment comprises preparing the untreated electrode 10A shown in Fig. 33. Here, the untreated electrode 10A comprises an untreated surface portion 11A partially having an untreated region 500 for forming the first region 110, the second region 120, the third region 130, and the fourth region 140, and having a first surface free energy, and an insulating layer 50 that is at least partially disposed on the untreated surface portion 11A, and includes an opening 52 formed in the untreated region 500 and passing through in the thickness direction. Here, the materials constituting the untreated electrode 10A and the insulating layer 50 can be selected from the same ranges as the materials constituting the electrode 10 and the insulating layer 50 in the electrode 10 and the sensor 1 of the various embodiments disclosed in this specification.

The method for manufacturing the electrode 10 according to this embodiment further includes a step of irradiating an annular fourth untreated region 540 that is disposed around the outer periphery of an untreated region 500 of the untreated surface portion 11A of the untreated electrode 10A, said annular fourth untreated region 540 being adjacent to the inner peripheral edge portion 55 of the opening 52 of the insulating layer 50 in plan view from a direction perpendicular to the untreated surface portion 11A, and an annular second untreated region 520 that is disposed further to the inside than the fourth untreated region 540, with a laser beam, thereby converting the fourth untreated region 540 into a fourth region 140 and converting the second untreated region 520 into a second region 120.

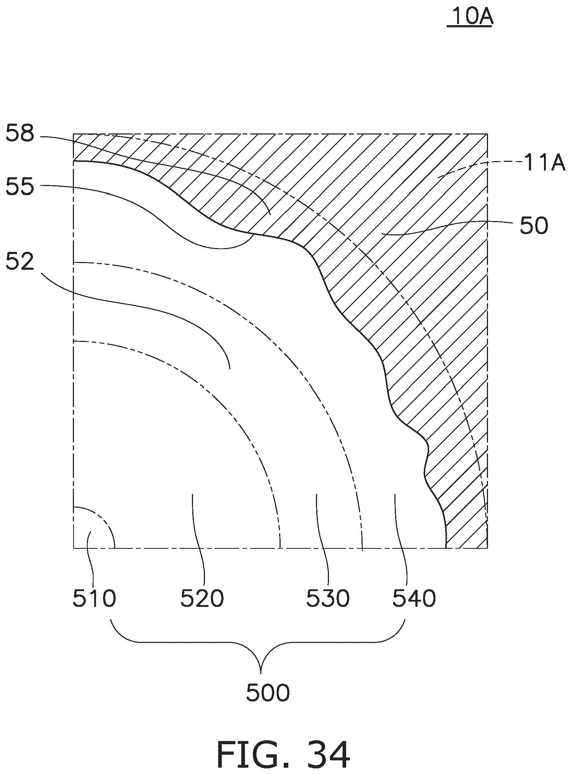

Fig. 34 schematically shows a detail view of the portion of the untreated electrode 10A shown in Fig. 33 that includes a boundary portion between the untreated region 500 of the untreated surface portion 11A and the insulating layer 50 around the opening 52 in plan view. In the illustrated example, the fine recesses and protrusions of the inner peripheral edge portion 55 of the opening 52 of the insulating layer 50 are shown in exaggerated form. The portion of the untreated region 500 that is surrounded by the second untreated region 520 is the first untreated region 510, and the portion between the second untreated region 520 and the fourth untreated region 540 is the third untreated region 530. That is, the untreated region 500 is made up of the first untreated region 510, the annular second untreated region 520 surrounding the first untreated region 510, the annular third untreated region 530 surrounding the second untreated region 520, and the annular fourth untreated region 540 surrounding the third untreated region 530, and the entire untreated region 500 has the first surface free energy.

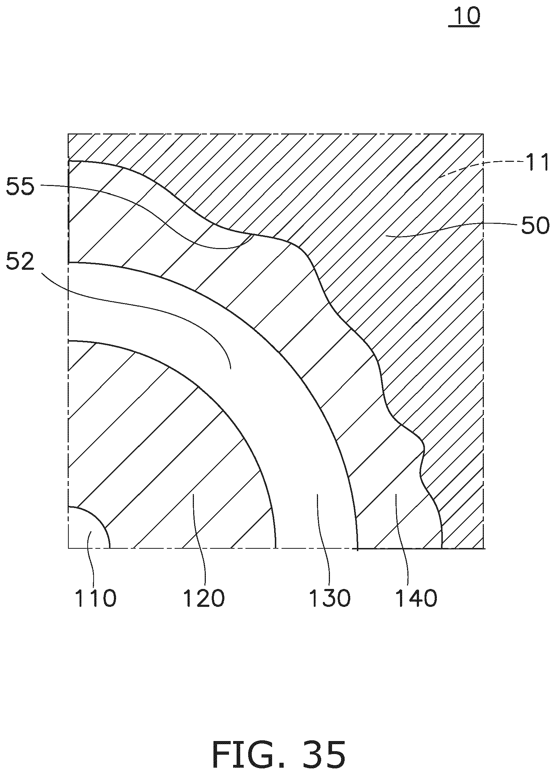

Fig. 35 schematically shows the portion of the electrode 10 corresponding to a portion of the untreated electrode 10A shown in Fig. 34 obtained by the irradiation with the laser beam converting the fourth untreated region 540 into the fourth region 140 and the second untreated region 520 into the second region 120. The irradiation with the laser beam here is a treatment in which the surface free energy of the second untreated region 520 and the fourth untreated region 540 is increased by scanning and irradiating with the laser beam, for example, to convert these into the second region 120 having the second surface free energy and the fourth region 140 having the fourth surface free energy, respectively. It is preferable for the first untreated region 510 and the third untreated region 530 of the untreated region 500 in the untreated electrode 10A not to be irradiated with the laser beam, so that these become the first region 110 and the third region 130 in the electrode 10 having the first surface free energy. In this case, the third region 130 has a third surface free energy that is the same as the first surface free energy. A preferred embodiment of the irradiating laser beam was already described above.

When the fourth untreated region 540 of the untreated region 500 of the untreated surface portion 11A shown in Fig. 34 is irradiated with a laser beam, it is preferable to irradiate the opening surrounding region 58 of the insulating layer 50 that surrounds the opening 52, which is adjacent to the outside of the fourth untreated region 540 in plan view from a direction perpendicular to the untreated surface portion 11A, with the laser beam in addition to the fourth untreated region 540. With this embodiment, even in the case where the inner peripheral edge portion 55 of the opening 52 of the insulating layer 50 has fine recesses and protrusions on the inside as shown in the drawings, it is possible to irradiate the fourth untreated region 540 of the untreated region 500 right up to the inner peripheral edge portion 55 with the laser beam to convert it into the fourth region 140. With the electrode 10 according to the embodiment shown in Figs. 30 and 31 and manufactured in this manner, the above-mentioned effect is especially likely to be obtained, wherein the second liquid composition applied inside the opening 52 of the insulating layer 50 can wet and spread out to the fourth outer peripheral edge portion 142 of the fourth region 140, and the protective film 40 obtained by drying the second liquid composition will have the designed surface area and will not readily peel off.

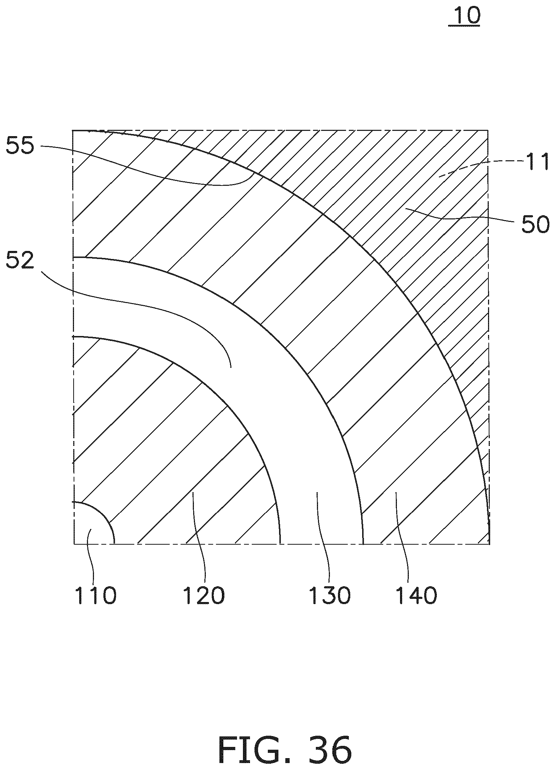

A more preferred embodiment of the embodiment in which the opening surrounding region 58 of the insulating layer 50 is irradiated with the laser beam in addition to the fourth untreated region 540 in the untreated region 500 of the untreated surface portion 11A shown in Fig. 34, further comprises a step of irradiating the opening surrounding region 58 of the insulating layer 50 with the laser beam to break up the insulating layer 50 in the opening surrounding region 58, and converting the portion of the untreated surface portion 11A that has been exposed by this breakup into the fourth region 140. Fig. 36 schematically shows a portion of the electrode 10 manufactured in this preferred embodiment. The fourth region 140 of the electrode 10 manufactured in this preferred embodiment is a region in which the surface free energy of the fourth untreated region 540 of the untreated region 500 and the portion where the opening surrounding region 58 of the insulating layer 50 has been broken up and exposed, out of the untreated surface portion 11A, has been raised to a fourth surface free energy by irradiation with a laser beam. With this preferred embodiment, since the fourth outer peripheral edge portion 142 of the fourth region 140 that defines the outer periphery of the protective film 40 and the inner peripheral edge portion 55 of the opening 52 of the insulating layer 50 are formed by laser beam irradiation, it is possible to define the outer periphery of the protective film 40 more accurately than when the insulating layer 50 having the opening 52 is formed by printing. Also, with this preferred embodiment, the second outer peripheral edge portion 122 of the second region 120 that defines the outer periphery of the reagent layer 30, the fourth outer peripheral edge portion 142 of the fourth region 140 that defines the outer periphery of the protective film 40, and the inner peripheral edge portion 55 of the opening 52 of the insulating layer 50 are all formed by irradiation with a laser beam. The method of this preferred embodiment manufactures an electrode 10 on which the reagent layer 30 and the protective film 40 can be formed more accurately and coaxially.

Working Examples

In the following experiments, operations in which no particular temperature is specified were performed at room temperature (21°C ±3°C).

Experiment 1

As shown in Fig. 7, the annular portion of the surface portion 11 of the electrode 10 was worked with a laser to raise the surface free energy and form the second region 120, with the interior of the second region 120 being termed the first region 110 and the exterior termed the third region 130. A reagent layer 30 was formed in the first region 110 and the second region 120 of the electrode 10 thus obtained to manufacture a sensor 1 equipped with a working electrode 2.

Substrate

A substrate with a thickness of 188 $\mu$m and made of polyethylene terephthalate, having the shape shown in Fig. 1 was used as the insulating substrate 20.

Electrode

A carbon paste was applied to the first surface 21 of the insulating substrate 20 and heated at 140°C for 1 hour, thereby forming two electrodes 10, a reference electrode conductive layer 301, a counter electrode 4, and wiring 5 electrically connected to each of these, which were each made of a carbon conductive layer with a thickness of 5 $\mu$m, in the shape shown in Fig. 1.

Laser Working

The surface free energy was raised by subjecting the annular portion of the surface portion 11 of the electrode 10 disposed on the substrate 20 to laser working to form the second region 120. The annular portion shown in Fig. 7, having a diameter M of 1.2 mm and a width N of 0.1 mm, 0.2 mm, or 0.3 mm, was worked by laser under the following conditions to form the second region 120 of the electrode 10 of a working example. As a comparative example, laser working was performed under the same conditions on a circular region with a diameter of 1.2 mm on the surface portion 11 of the electrode 10 disposed on the substrate 20.

Table 1

| Working Conditions | |
|---|---|
| Laser | UV |
| Wavelength (nm) | 355 |
| Laser output (mW) | 110 |
| Scan Speed (mm/s) | 500 |
| Frequency (kHz) | 200 |

The surface free energy (dispersive component, polar component) of the surface portion 11 of the electrode 10 was measured before laser working and after a certain amount of time had elapsed after laser working. The surface free energy was calculated by using a contact angle meter to measure the contact angle (drop amount of 1 $\mu$L, measured 2 seconds after dropping) with respect to water and diiodomethane at 20°C and a relative humidity of 40%, and using the simultaneous equations consisting of the above formulas (1), (2), and (3).

The surface free energy (dispersive component, polar component) of the second region 120 of the electrode 10 of a working example formed by laser working and the circular region of the electrode in a comparative example at various elapsed times after laser working is shown in the table below. The surface free energy at zero minutes of elapsed time was the surface free energy of the surface portion 11 of the electrode 10 before laser working, and was considered to be the surface free energy of the first region 110 and the third region 130.

Table 2

| Elapsed time (min) | | Dispersive component γsd (mN/m) | Polar component γsp (mN/m) | Surface free energy γs (mN/m) |
|---|---|---|---|---|
| 0 | | 44.92 | 2.9 | 47.82 |
| 10 | | 47.13 | 2.79 | 49.92 |
| 15 | | 47.14 | 5.71 | 52.85 |
| 30 | | 47.11 | 3.33 | 50.44 |
| 60 | 1h | 47.22 | 3.85 | 51.07 |
| 120 | 2h | 47.64 | 5.79 | 53.43 |
| 180 | 3h | 48.47 | 5 | 53.47 |
| 240 | 4h | 48.5 | 6.44 | 54.94 |
| 300 | 5h | 47.86 | 8.26 | 56.12 |
| 360 | 6h | 47.44 | 8.35 | 55.79 |
| 1440 | 24h | 48.48 | 11.8 | 60.28 |
| 2880 | 2d | 48.47 | 14.69 | 63.16 |

First Liquid Composition

As a first liquid composition for forming a reagent layer, an aqueous solution containing a carbon black dispersion, hydroxypropyl cellulose, a polymer-bound mediator, lactate oxidase, polyimidazole, poly-L-lysine, and a crosslinking agent in water was prepared.

Formation of Reagent Layer

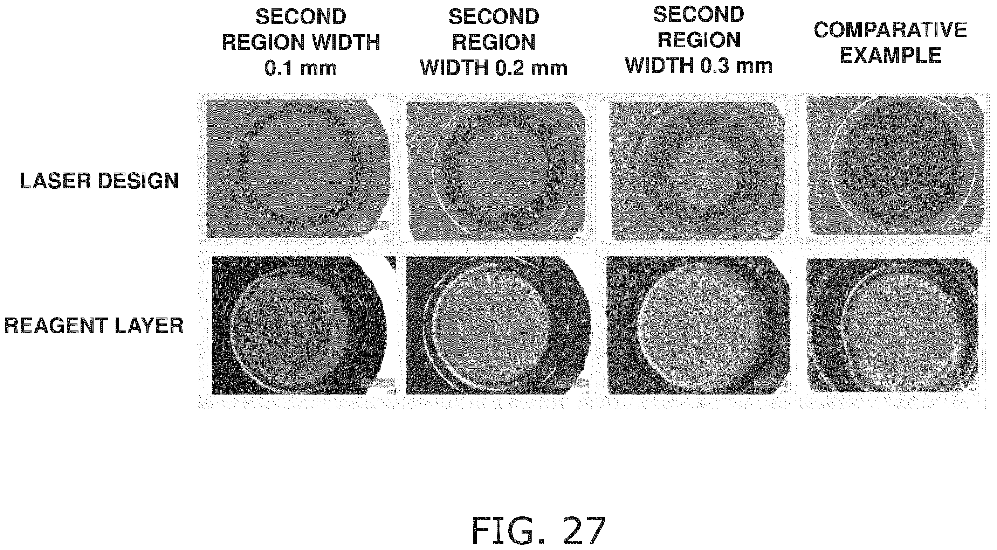

24 hours after the laser working, a droplet was formed by dropping 0.65 mg of the first liquid composition onto the center of the first region 110 of the electrode 10 of a working example, which had a first region 110 and a laser-worked annular second region 120 of various widths surrounding the first region 110, and the droplet was then dried to form a reagent layer 30 containing lactate oxidase and a mediator, thereby producing a sensor 1 equipped with a working electrode 2.
Meanwhile, 24 hours after the laser working, a droplet was formed by dropping 0.65 mg of the first liquid composition onto the center of the circular region of the electrode of a comparative example, which had a laser-worked circular region, and the droplet was then dried to form a reagent layer containing lactate oxidase and a mediator.
A photograph of the reagent layer formed on each electrode is shown in Fig. 27. In all of the electrodes 10 of the working examples, the reagent layer 30 was formed only in the first region 110 and the second region 120, and the outer peripheral edge portion 31 of the reagent layer 30 was located on the second outer peripheral edge portion 122 of the second region 120.
Meanwhile, in 8 of the 12 samples of the comparative example electrodes, the first liquid composition applied to the circular region overflowed outside the circular region, resulting in an irregularly shaped reagent layer after drying.

Experiment 2

Using the same material and performing laser working in the same procedure as in Experiment 1, an annular second region 120 having a diameter M of 1.2 mm and a width N of 0.3 mm was formed on the surface portion 11 of the electrode 10 as shown in Fig. 7, with the first region 110 on the inside and the third region 130 on the outside thereof.
A nozzle was brought to 0.5 mm away from the center of the first region 110 of this electrode 10, and 0.65 mg of the same first liquid composition as in Experiment 1 was dropped from the nozzle, and then the nozzle was moved away and the droplet dried to form a reagent layer 30 containing lactate oxidase and a mediator.
The formation of a reagent layer 30 was conducted ten times, and in five of these, when the nozzle was moved away, the first liquid composition clung to the nozzle and pulled away from the electrode 10, making coating impossible.

Experiment 3

Using the same material and performing laser working in the same procedure as in Experiment 1, an electrode 10 was produced that had the shape shown in Fig. 14 and included a first region 110 including a center region 112 (laser-worked portion) and a surrounding region 115 (unworked portion), an annular second region 120 (laser-worked portion), and a third region 130 on the outside of the second region 120. The diameter W of the center region 112 shown in Fig. 14 was 0.25 mm, the diameter M of the second region 120 was 1.2 mm, and the width N of the second region 120 was 0.25 mm.

As in Experiment 2, the nozzle was brought to a distance of 0.5 mm away from the center region 112 of the first region 110 of the electrode 10, and 0.65 mg of the same first liquid composition as in Experiments 1 and 2 was dropped from the nozzle, after which the nozzle was moved away and the droplet was dried to form a reagent layer 30 containing lactate oxidase and a mediator.

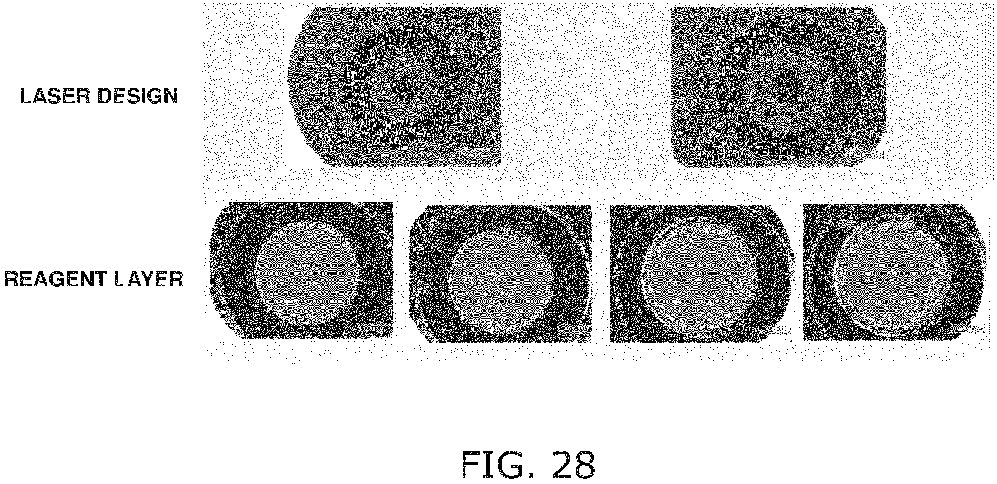

The application and drying of the first liquid composition was conducted about 200 times, and each time the first liquid composition was applied normally. The reagent layer 30 obtained by drying the applied first liquid composition was formed only in the first region 110 and the second region 120, and the outer peripheral edge portion 31 of the reagent layer 30 was accurately positioned on the second outer peripheral edge portion 122 of the second region 120. Fig. 28 shows a photograph of a portion of the reagent layer 30 thus formed.

Experiment 4

Using the same materials and conditions as in Experiment 1, laser working was performed under the same conditions to form an annular second region 120 having a diameter M of 1.2 mm and a width N of 0.1 mm or 0.3 mm as shown in Fig. 7 on the surface portion 11 of the electrode 10 disposed on the first surface 21 of the insulating substrate 20, the inside of which was termed the first region 110 and the outside the third region 130, just as in Experiment 1. As in Experiment 1, laser working was performed under the same conditions on a circular region having a diameter of 1.2 mm of the surface portion 11 of the electrode 10 disposed on the substrate 20, as a comparative example.

A liquid containing a carbon black dispersion, a polymer-bound mediator, lactate oxidase, polyimidazole, and ε-poly-L-lysine in ultrapure water (Milli-Q (registered trademark) water) was prepared as a first liquid composition for forming a reagent layer.

24 hours after the laser working, a droplet was formed by dropping the first liquid composition, in the amount discussed below, onto the center of the first region 110 of the electrode 10 of a working example, which had a first region 110 and a laser-worked annular second region 120 of various widths that surrounded the first region 110, after which the droplet was dried to form a reagent layer 30 for detecting lactic acid. Meanwhile, 24 hours after the laser working, the first liquid composition was dropped in the amount described below onto the center of the circular region of the electrode of the comparative example having a laser-worked circular region to form a droplet, which was then dried to form a reagent layer for detecting lactic acid.

In this test, the first liquid composition was used in amounts gradually increasing by 0.05 g each time from 0.40 g as the first liquid composition to be dropped in order to form the above-mentioned reagent layer. The maximum holding amount of the first liquid composition at which a reagent layer could be formed in a true circular shape without overflowing in the first region 110 and the second region 120 of the electrode 10 of each working example or in the circular region of the electrode of the comparative example (the maximum holding amount of the first liquid composition at which a reagent layer with a true circular shape could be formed without overflowing in 10 tests) was checked. As a result, it was confirmed that the maximum amount of the first liquid composition was 0.75 mg for the electrode 10 in the working example having the annular second region 120 with a diameter M of 1.2 mm and a width N of 0.1 mm shown in Fig. 7, 0.70 mg for the electrode 10 in the working example having the annular second region 120 with a diameter M of 1.2 mm and a width N of 0.3 mm, and 0.60 mg for the electrode in the comparative example having a circular region with a diameter of 1.2 mm. That is, it was confirmed that forming in the center a first region 110 having a first surface free energy with low wettability by the first liquid composition, and forming a second region 120 around this having a second surface free energy with high wettability by the first liquid composition, allows a larger amount of the first liquid composition to be held, and that the smaller is the width N of the second region 120, the larger is the amount of the first liquid composition that can be held.

Experiment 5

A sensor 1 having the configuration shown in Fig. 5 was produced. An overview of the materials and the manufacturing method used for the sensor 1 having the configuration shown in Fig. 5 is given below. Of the two working electrodes 2, one was used as a working electrode for detecting lactic acid, and the other was used as a working electrode for detecting glucose. Since lactic acid was measured in the measurement test described later, only the manufacturing method of the working electrode for detecting lactic acid will be described below.

Substrate

A substrate made of polyethylene terephthalate, having a thickness of 188 μm, and having the shape shown in Fig. 1 and other figures, was used as the insulating substrate 20.

Conductive Layer

Carbon paste was applied to the first surface 21 of the insulating substrate 20, and this coating was heated at 140°C for 1 hour to form the electrode 10, the reference electrode conductive layer 301, the counter electrode 4, and the wiring 5 electrically connected to these, each of which had the shape shown in Fig. 1 and were made of a carbon conductive layer with a thickness of 5 μm.

Laser working

Under the conditions described in Experiment 1, the second region 120 was formed by subjecting the annular portion of the surface portion 11 of the electrode 10 disposed on the substrate 20, having a diameter M of 1.4 mm and a width N of 0.3 mm as shown in Fig. 7, to laser working to raise the surface free energy. Of the surface portion 11 of the electrode10, the portion inside second region 120 is the first region 110, and the portion outside the second region 120 is the third region 130.

Insulating Layer

Then, as shown in Fig. 3, an insulating layer 50 made of a fluororesin was laminated on the first surface 21 of the substrate 20 on which the carbon conductive layer was disposed.
The opening 52 formed in the first region 110, the second region 120, and the third region 130 of the insulating layer 50 was circular, with a diameter of 2.0 mm. The reference electrode opening 53 was circular, with a diameter of 1.0 mm, and the counter electrode opening 54 was rectangular, measuring $2.7 \times 2.65$ mm.

Reagent Layer

24 hours after the laser working, the composition described in Experiment 4, containing lactate oxidase, was dropped in amounts of 0.40 mg, 0.45 mg, 0.50 mg, 0.55 mg, and 0.60 mg onto the center of the first region 110 in the opening 52 of the insulating layer 50 to form a droplet in the first region and the second region, and these were then dried to form a reagent layer 30 for detecting lactic acid (see Fig. 4).

Protective Film

Ethanol (produced by FUJIFILM Wako Pure Chemical Industries) and a sodium hydroxide aqueous solution (produced by FUJIFILM Wako Pure Chemical Industries) were added to a Nafion (registered trademark) dispersion (produced by Sigma-Aldrich) to adjust the pH of the dispersion (for neutralization of the cation exchange groups), the precipitate was dissolved using a vortex mixer, and the resulting liquid was adjusted to 9 wt% to prepare a second liquid composition A.
The following reagents were mixed with ethanol so as to give the following final concentrations, and the mixture was allowed to react for about 1 hour to prepare a second liquid composition B.

- P4VP-tBuMA (poly-4-vinylpyridine Mn: 74,000, poly-tert-butyl methacrylate Mn: 87,000, Mw/Mn: 1.16, produced by NARD), final concentration of 3.55 wt%
- random copolymer of tripropylene glycol methyl ether methacrylate-styrene-4-vinylpyridine (produced by NARD), final concentration of 4.45 wt%
- PEGDGE (poly(ethylene glycol) diglycidyl ether, Mn: 1,000, produced by Sigma-Aldrich), 0.9 wt%

0.6 mg of the second liquid composition A was dropped into the opening 52 in the insulating layer 50 to form a droplet, which was then dried to form a first layer of the protective film 40, and then 0.6 mg of the second liquid composition B was dropped into the opening 52 to form a droplet, which was then dried to form a second layer of the protective film 40, thereby producing a two-layer protective film 40. The working electrode 2 having the cross-sectional structure shown in Fig. 11 was thus completed (although not depicted, the protective film 40 has a two-layer structure).

Reference Electrode

A silver/silver chloride paste was applied onto the reference electrode conductive layer 301 in the reference electrode opening 53 of the insulating layer 50, and heated at 140°C for 1 hour to form a silver/silver chloride layer 302. Then, a reference electrode protective film 303 was disposed on the silver/silver chloride layer 302 to form a reference electrode 3 having the cross-sectional structure shown in Fig. 12.

Storage at High Temperature

The sensor 1 produced as above was stored at 60°C for 2 weeks after production and then used to measure lactic acid concentration.

Continuous Measurement of Lactic Acid Concentration

Measurement Media

DMEM, no glucose (Gibco 11966025)
penicillin-streptomycin-amphotericin B suspension ($\times$ 100) (antibiotic-antifungal solution) (FUJIFILM Wako Pure Chemical Industries, 161-23181)
sodium pyruvate (100 mM) (Gibco 11360070)
Using the above reagents, a DMEM culture medium containing a penicillin-streptomycin-amphotericin B suspension at a final concentration of 1% (v/v) and sodium pyruvate at a final concentration of 1 mM was prepared.

Using the DMEM culture medium, measurement media were prepared as shown below.

Medium 1: DMEM medium adjusted to a final lactic acid concentration of 6 mM
Medium 2: DMEM medium adjusted to a final lactic acid concentration of 12 mM
Medium 3: DMEM medium adjusted to a final concentration of about 9% (v/v) FBS (fetal bovine serum, Gibco 16140071) and a final lactic acid concentration of 16 mM
Blank medium: DMEM medium adjusted to a final lactic acid concentration of 10 mM

A voltage of 100 mV was applied to the working electrode 2 immersed in the measurement medium, relative to the reference electrode 3 (Ag/AgCl), and the current between the working electrode 2 and the counter electrode 4 was measured continuously for about 12 days (about 288 hours). This measurement was carried out in a $CO_2$ incubator at 5% $CO_2$ and a temperature of 37°C.
Measurement test (N = 3): The above-mentioned media 1 to 3 were used as the measurement medium, and were replaced over time in the following sequence to carry out the above-mentioned measurements.

Medium 1: about 24 hours $\rightarrow$ Medium 2: about 24 hours $\rightarrow$ Medium 3: about 72 hours $\rightarrow$
Medium 1: about 9 hours $\rightarrow$ Medium 2: about 15 hours $\rightarrow$ Medium 3: about 72 hours $\rightarrow$
Medium 3: about 72 hours

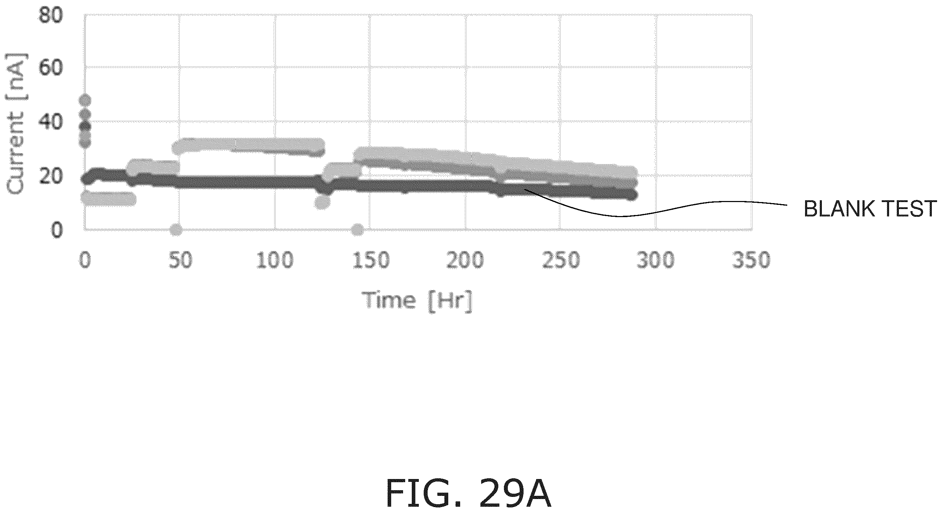

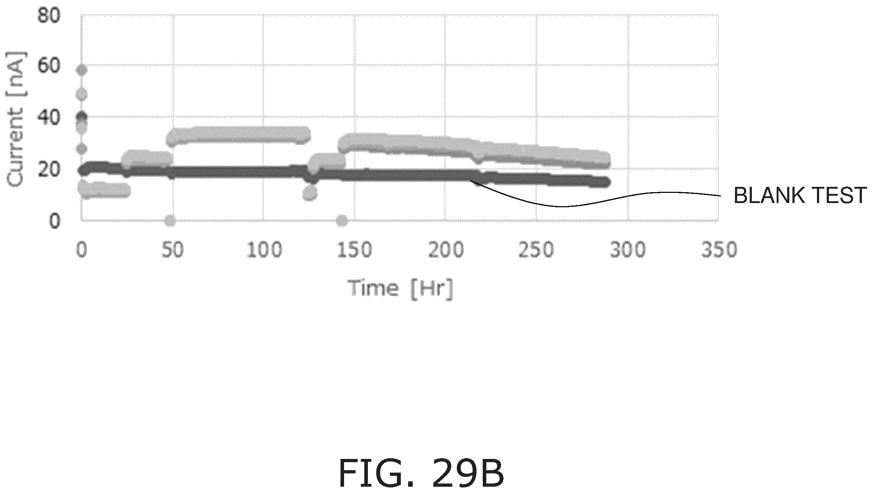

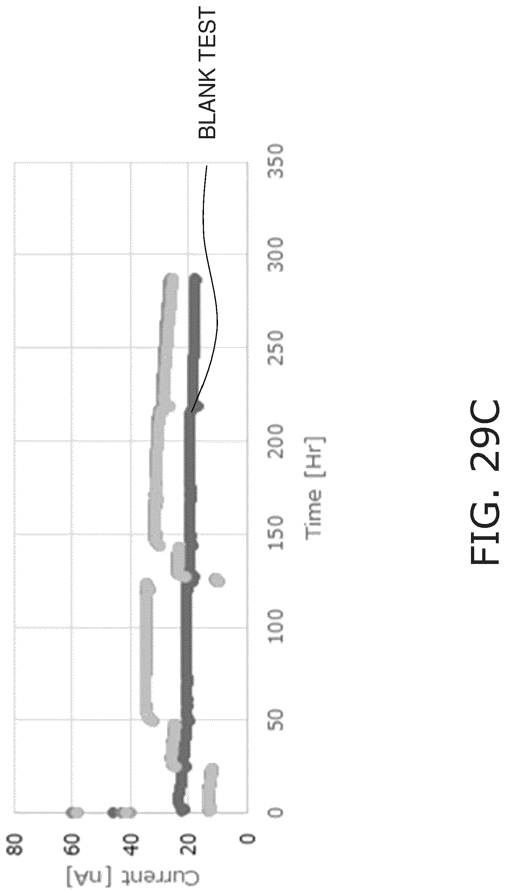

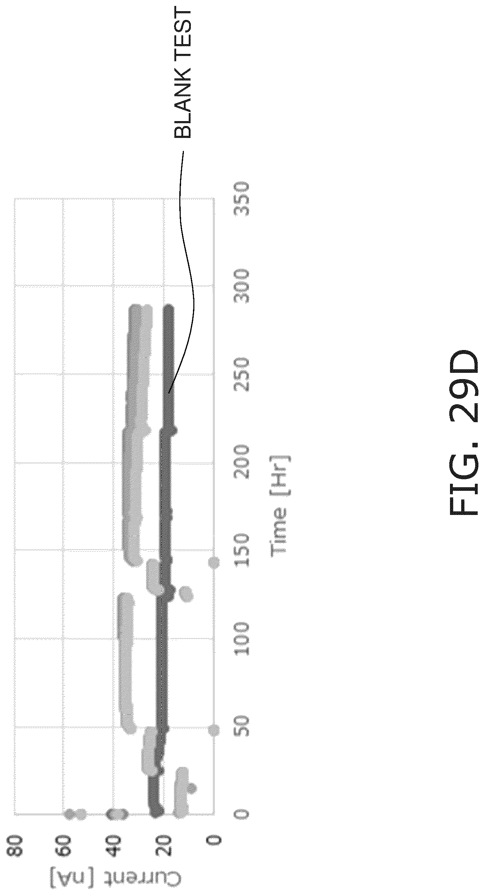

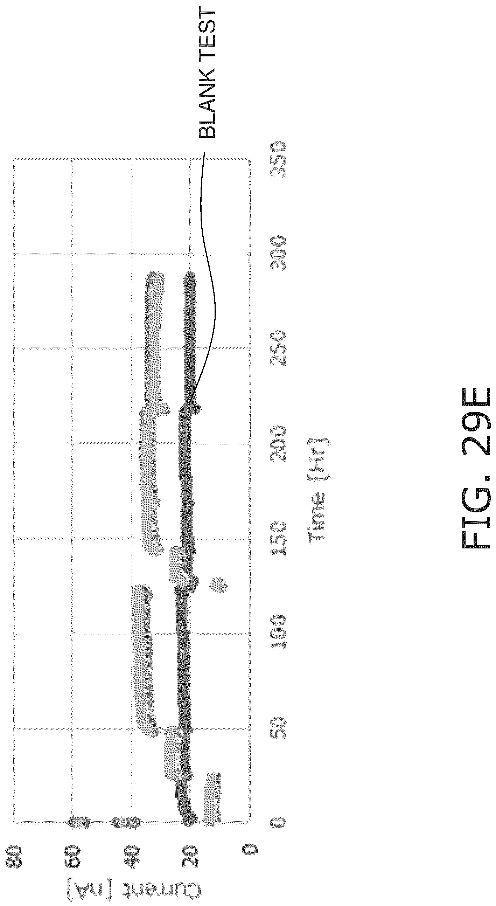

Blank test (N = 1): The blank medium was used as the measurement medium, and was replaced with a fresh medium at the same timing as in the above measurement test, and the above measurements were carried out for about 288 hours.
The results of measuring the current between the working electrode 2 for measuring lactic acid and the counter electrode 4 are shown in Figs. 29A to 29E. Fig. 29A shows the current value for the sensor 1 in which the amount of lactate oxidase-containing first liquid composition having the composition described in Experiment 4 used to prepare the reagent layer 30 was applied was 0.40 mg, Fig. 29B shows the current value for the sensor 1 in the case where it was 0.45 mg, Fig. 29C shows the current value for the sensor 1 in the case where it was 0.50 mg, Fig. 29D shows the current value for the sensor 1 in the case where it was 0.55 mg, and Fig. 29E shows the current value for the sensor 1 in the case where it was 0.60 mg.
The decay rate of the current value of the sensor 1 after 12 days was 37.8% in the case where the applied amount of the lactate oxidase-containing first liquid composition of the composition described in Experiment 4 used to prepare the reagent layer 30 was 0.40 mg (Fig. 29A), 29.0% in the case where it was 0.45 mg (Fig. 29B), 20.0% in the case where it was 0.50 mg (Fig. 29C), 17.0% in the case where it was 0.55 mg (Fig. 29D), and 11.0% in the case where it was 0.60 mg (Fig.

29E). These results indicate that the greater is the amount of reagent held in the reagent layer 30, the longer the analyte can be continuously measured. It was suggested that the sensor 1 having the electrode 10 disclosed in this specification is suitable for long-term continuous measurement of an analyte because a larger amount of reagent can be held in the reagent layer 30.

Experiment 6

An electrode 10 having the configuration shown in Figs. 30 and 31 was produced, and a reagent layer 30 and a protective film 40 were further formed to produce the working electrode 2 shown in Fig. 32. However, in this Experiment 6, unlike the examples shown in Figs. 30 to 32, instead of the annular second region 120 encompassing the circular first region 110, no first region 110 was provided, a circular second region 120 entirely having a second surface free energy was formed, and the reagent layer 30 was disposed in the circular second region 120.

Carbon paste was applied onto the first surface 21 of an insulating substrate 20 made of polyethylene terephthalate and having a thickness of 188 $\mu$m, and this coating was heated at 140°C for 1 hour to form an untreated electrode 10A made of a carbon conductive layer having a thickness of 5 $\mu$m.

Then, an insulating layer 50 made of a fluororesin was laminated by screen printing on the first surface 21 of the substrate 20 on which the untreated electrode 10A was disposed. The thickness of the insulating layer 50 on the untreated electrode 10A was 5 $\mu$m. The opening 52 in the insulating layer 50 formed on the untreated region 500 of the untreated surface portion 11A of the untreated electrode 10A was circular, with a diameter (inside diameter) of 2.0 mm.

In Experiment 6-1, under the conditions described in experiment 1, a circular second untreated region 520 having a diameter of 1.4 mm from the center of the untreated region 500 of the untreated surface portion 11A of the untreated electrode 10A in the opening 52 of the insulating layer 50, and an annular fourth untreated region 540 adjacent to the inner peripheral edge portion 55 of the opening 52 of the insulating layer 50 and located on the outer periphery at a position 1.75 mm and more away from the center of the untreated region 500, were irradiated with a laser beam, thereby converting the circular second untreated region 520 into the circular second region 120, and the annular fourth untreated region 540 into the annular fourth region 140. At this point, the opening surrounding region 58 of the insulating layer 50 adjacent to the outside of the fourth untreated region 540 was also irradiated with the laser beam. The portion between the second region 120 and the fourth region 140 after the laser beam irradiation is the third region 130. Since the laser beam irradiation was carried out under the same conditions as in Experiment 1, the surface free energy $\gamma$s of the untreated region 500 of the untreated surface portion 11A of the untreated electrode 10A, and the third region 130 of the electrode 10 was 47.82 mN/m (the dispersive component $\gamma$sd was 44.92 mN/m, and the polar component $\gamma$sp was 2.90 mN/m). The surface free energy $\gamma$s of the second region 120 and the fourth region 140 of the electrode 10 24 hours after the laser beam irradiation was 60.28 mN/m (the dispersive component $\gamma$sd was 48.48 mN/m, and the polar component $\gamma$sp was 11.80 mN/m). Fig. 37 (upper right part) shows a photograph of the electrode 10 after the laser beam irradiation in Experiment 6-1.

In experiment 6-2, under the conditions described in Experiment 1, out of the untreated region 500 of the untreated surface portion 11A of the untreated electrode 10A in the opening 52 of the insulating layer 50, only a circular second untreated region 520 having a diameter of 1.4 mm from the center of the untreated region 500 was irradiated with the laser beam to convert this part into the circular second region 120. The portion more to the outside than the second region 120 in the opening 52 of the insulating layer 50 after the laser beam irradiation is the third region 130. The surface free energies of the second region 120 and the third region 130 after the laser beam irradiation are the same as those in Experiment 6-1. Fig. 37 (upper left part) shows a photograph of the electrode 10 after the laser beam irradiation in Experiment 6-2.

Reagent Layer

24 hours after the laser working, 0.5 mg of the lactate oxidase-containing first liquid composition having the composition described in Experiment 4 was dropped onto the center of the second region 120 in the opening 52 of the insulating layer 50 of the electrode 10 of Experiment 6-1 or Experiment 6-2 to form a droplet in the second region 120, which was then dried to form a reagent layer 30 for detecting lactic acid (see Fig. 32).

Protective Film

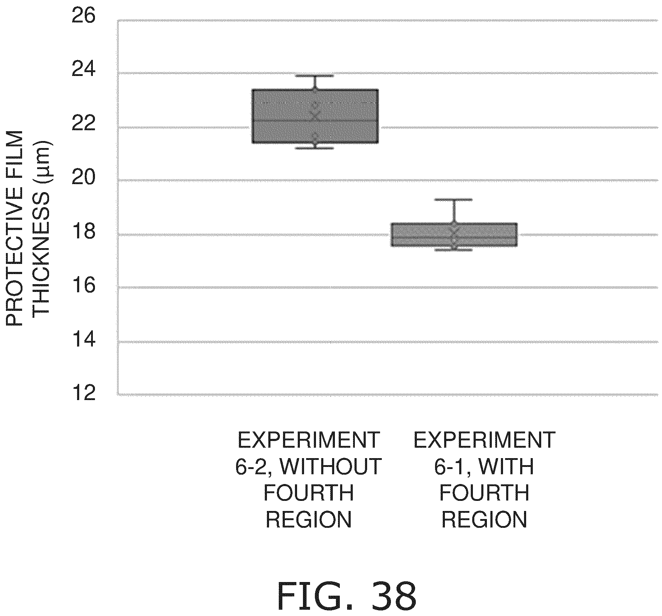

After the reagent layer 30 was formed, 0.6 mg of the second liquid composition A described in Experiment 5 was dropped into the opening 52 of the insulating layer 50 of the electrode 10 of Experiment 6-1 or Experiment 6-2 to form a droplet, which was then dried to form a first layer of the protective film 40, and then 0.6 mg of the second liquid composition B described in Experiment 5 was dropped into the opening 52 of the insulating layer 50 of the electrode 10 of Experiment 6-1 or Experiment 6-2 to form a droplet, which was then dried to form a second layer of the protective film 40, thereby producing a protective film 40 with a two-layer structure. This completed a working electrode 2 with the cross-sectional structure shown in Fig. 32 (although not depicted, the protective film 40 has a two-layer structure). The thickness of the dried protective film 40 on the electrode 10 of Experiment 6-1 or Experiment 6-2 was measured on the basis of the difference in height measured before and after the formation of the protective film 40 using a white light interferometer displacement sensor. The thickness measurement results are shown in Fig. 38.

Shape of Protective Film

Fig. 37 (lower left part) shows a photograph of the working electrode 2 in which the electrode 10 of Experiment 6-2 is used. With the working electrode 2 in which the electrode 10 of Experiment 6-2 is used, the outside diameter of the protective film 40 was 1.88 mm, which was smaller than the designed value for the inside diameter of the opening 52 of the insulating layer 50. Also, as shown in Fig. 38, it was confirmed that the thickness of the protective film 40 in the working electrode 2 featuring the electrode 10 of Experiment 6-2 was about 22 $\mu$m, which was greater than the case where the electrode 10 of Experiment 6-1 was used, and the standard deviation was about twice as large. These results suggest that the inner peripheral edge portion 55 of the opening 52 of the insulating layer 50 formed by screen printing has fine recesses and protrusions in plan view, and that with the electrode 10 of Experiment 6-2, the second liquid compositions A and B for producing the protective film 40 applied to the opening 52 of the insulating layer 50 could not sufficiently adhere to the inner peripheral edge portion 55 of the opening 52 of the insulating layer 50 and to the third outer peripheral edge portion 132 of the third region 130.

Fig. 37 (lower right part) shows a photograph of the working electrode 2 in which the electrode 10 of Experiment 6-1 was used. With the working electrode 2 in which the electrode 10 of Experiment 6-1 was used, the outside diameter of the protective film 40 matched 2.0 mm, which was the design value for the inside diameter of the opening 52 of the insulating layer 50. Also, as shown in Fig. 38, it was confirmed that the thickness of the protective film 40 in the working electrode 2 in which the electrode 10 of Experiment 6-1 was used was about 18 $\mu$m, which was less than in the case of using the electrode 10 of Experiment 6-2, and the standard deviation was smaller. With the electrode 10 of Experiment 6-1, the outer peripheral edge of the bottom inside the opening 52 of the insulating layer 50 is the fourth region 140 that has the fourth surface free energy, which suggests that the second liquid compositions A and B for producing the protective film 40 were able to spread over the entire opening 52 of the insulating layer 50, and that because a part of insulating layer 50 corresponding to the fine recesses and protrusions of the inner peripheral edge portion 55 of the opening 52 were destroyed by laser beam irradiation and the exposed untreated surface portion 11A was converted into the fourth region 140, the second liquid compositions A and B were able to adhere sufficiently to the inner peripheral edge portion 55 of the opening 52 of the insulating layer 50, and therefore the protective film 40 could be formed over the entire opening 52 of the insulating layer 50.

This specification includes disclosures related to the following appendices 1 to 20.

Appendix 1 An electrode to be disposed on an insulating substrate, said electrode comprising:

a surface portion that is located on the opposite side from the substrate when the electrode is disposed on the substrate;
a first region that is formed on the surface portion, includes a first outer peripheral edge portion, and at least a portion thereof has a first surface free energy;
a second region that is formed on the surface portion, surrounds the first region, includes a second inner peripheral edge portion in contact with the first outer peripheral edge portion of the first region, and a second outer peripheral edge portion located more to the outside than the second inner peripheral edge portion, and has a second surface free energy that is greater than the first surface free energy; and
a third region that is formed on the surface portion, surrounds the second region, includes a third inner peripheral edge portion in contact with the second outer peripheral edge portion of the second region, and has a third surface free energy that is less than the second surface free energy.

Appendix 2
The electrode according to Appendix 1,
wherein the entire first region has the first surface free energy.
Appendix 3
The electrode according to Appendix 1,
wherein the first region includes:

a center region that is disposed in the interior of the first region, includes an outer peripheral edge portion located more to the inside than the first outer peripheral portion of the first region, and has a surface free energy greater than the first surface free energy; and

a surrounding region that surrounds the center region, includes an inner peripheral edge portion in contact with the outer peripheral edge portion of the center region, and the first outer peripheral edge portion, and has the first surface free energy.

Appendix 4
The electrode according to Appendix 3,
wherein the first region further includes:
a connecting region that extends through the surrounding region, from part of the outer peripheral edge portion of the center region to part of the second inner peripheral edge portion of the second region, which is opposite via the surrounding region, and has a surface free energy greater than the first surface free energy.

Appendix 5
The electrode according to any of Appendices 1 to 4,

wherein the third region further includes a third outer peripheral edge portion located more to the outside than the third inner peripheral edge portion, and
there is further provided a fourth region that is formed on the surface portion, surrounds the third region, includes a fourth inner peripheral edge portion in contact with the third outer peripheral edge portion of the third region, and a fourth outer peripheral edge portion located more to the outside than the fourth inner peripheral edge portion, and has a fourth surface free energy greater than the third surface free energy.

Appendix 6
The electrode according to Appendix 5,
further comprising an insulating layer, at least part of which is disposed on the surface portion, and that includes an opening formed in the first region, the second region, the third region, and the fourth region, having an inner peripheral edge portion that in contact with the fourth outer peripheral edge portion of the fourth region in plan view from a direction perpendicular to the surface portion, and passing through in the thickness direction.

Appendix 7
The electrode according to any of Appendices 1 to 5,
further comprising an insulating layer, at least part of which is disposed on the surface portion, and that includes an opening formed in the first region, the second region, and the third region, and passing through in the thickness direction.

Appendix 8
An electrode to be disposed on an insulating substrate, said electrode comprising:

a surface portion that is located on the opposite side from the substrate when the electrode is disposed on the substrate;
a first region that is formed on the surface portion, includes a first outer peripheral edge portion, and at least a portion thereof has a first surface free energy;
a second region that is formed on the surface portion, surrounds the first region, includes a second inner peripheral edge portion in contact with the first outer peripheral edge portion of the first region, and a second outer peripheral edge portion located more to the outside than the second inner peripheral edge portion, and has a second surface free energy that is greater than the first surface free energy; and
an insulating layer, at least a portion of which is disposed on the surface portion, and that includes an opening formed in the first region and the second region, having an inner peripheral edge portion that defines the second outer peripheral edge portion of the second region, and passing through in the thickness direction.

Appendix 9
The electrode according to Appendix 8,
wherein the entire first region has the first surface free energy.

Appendix 10
The electrode according to Appendix 8,
wherein the first region includes:

a center region that is disposed within the first region, includes an outer peripheral edge portion located more to the inside than the first outer peripheral portion of the first region, and has a surface free energy greater than the first surface free energy; and
a surrounding region that surrounds the center region, includes an inner peripheral edge portion in contact with the outer peripheral edge portion of the center region, and the first outer peripheral edge portion, and has the first surface free energy.

Appendix 11

The electrode according to Appendix 10,

wherein the first region further includes:

a connecting region that extends through the surrounding region, from part of the outer peripheral edge portion of the center region to part of the second inner peripheral edge portion of the second region, which is opposite via the surrounding region, and has a surface free energy greater than the first surface free energy.

Appendix 12

A sensor, comprising:

the electrode according to any of Appendices 1 to 11; and

a reagent layer that is disposed in the first region and the second region of the electrode, and includes an outer peripheral edge portion located on the second outer peripheral edge portion of the second region, and a reagent participating in an oxidation-reduction reaction.

Appendix 13

The sensor according to Appendix 12,

further comprising a protective film that covers the reagent layer.

Appendix 14

The sensor according to Appendix 12 or 13,

further comprising an insulating substrate on which the electrode is disposed.

Appendix 15

A method manufacturing a sensor comprising:

the electrode according to any of Appendices 1 to 11; and

a reagent layer that is disposed in the first region and the second region of the electrode, and includes an outer peripheral edge portion located on the second outer peripheral edge portion of the second region, and a reagent participating in an oxidation-reduction reaction,

said method comprising:

coating the first region and the second region of the electrode with a first liquid composition containing the reagent in a first solvent, and then drying the first liquid composition to form the reagent layer.

Appendix 16

The method according to Appendix 15,

wherein the first solvent includes water and/or an alcohol.

Appendix 17

A method for manufacturing a sensor comprising:

the electrode according to any of Appendices 1 to 11;

a reagent layer that is disposed in the first region and the second region of the electrode, and includes an outer peripheral edge portion located on the second outer peripheral edge portion of the second region, and a reagent participating in an oxidation-reduction reaction; and

a protective film that covers the reagent layer,

said method comprising:

coating the first region and the second region of the electrode with a first liquid composition containing the reagent in a first solvent, and then drying the first liquid composition to form the reagent layer, and

coating the surface portion of the electrode with a second liquid composition containing a protective film component in a second solvent so as to cover the reagent layer, then drying the second liquid composition to form the protective film.

Appendix 18

The method according to Appendix 17,

wherein the electrode is the electrode according to Appendix 5 or 6,

the protective film is disposed in the first region, the second region, the third region, and the fourth region of the electrode so as to cover the reagent layer, and includes an outer peripheral edge portion located on the fourth outer peripheral edge portion of the fourth region, and

the formation of the protective film comprises coating the first region, the second region, the third region, and the fourth region of the electrode with the second liquid composition so as to cover the reagent layer, and then drying the second liquid composition.

Appendix 19

A method for manufacturing the electrode according to Appendix 6, comprising the steps of:
preparing an untreated electrode including:

an untreated surface portion having an untreated region for forming the first region, the second region, the third region, and the fourth region in a part of the untreated surface portion, and having the first surface free energy, and an insulating layer, at least part of which is disposed on the untreated surface portion, and that includes an opening formed in the untreated region and passing through in the thickness direction; and
irradiating, out of the untreated region of the untreated surface portion in the untreated electrode, an annular fourth untreated region disposed on the outer periphery of the untreated region adjacent to the inner peripheral edge portion of the opening in the insulating layer and an annular second untreated region disposed further to the inside than the fourth untreated region in a plan view from a direction perpendicular to the untreated surface portion, with a laser beam, thereby converting the fourth untreated region into the fourth region, and converting the second untreated region into the second region.

Appendix 20

The method according to Appendix 19, the irradiation of the fourth untreated region of the untreated region with the laser beam further includes irradiating an opening surrounding region of the insulating layer that surrounds the opening, which region is adjacent to the outside of the fourth untreated region in plan view from a direction perpendicular to the untreated surface portion, with the laser beam.

Appendix 21

The method according to Appendix 20, wherein the irradiation of the opening peripheral region of the insulating layer with the laser beam further includes irradiating the opening surrounding region of the insulating layer with the laser beam to break up the insulating layer in the opening surrounding region and convert the portion of the untreated surface portion that is exposed by this breakup into the fourth region.

Appendix 22

In the electrodes according to Appendices 1 to 11, the first outer peripheral edge portion of the first region and the second inner peripheral edge portion and second outer peripheral edge portion of the second region are preferably circular. In the electrodes according to Appendices 1 to 10, more preferably, in the case where one or more members selected from among the third inner peripheral edge and third outer peripheral edge of the third region, and the fourth inner peripheral edge and fourth outer peripheral edge of the fourth region are present, it is even more preferable for these to be circular.

REFERENCE SIGNS LIST

1: sensor
2: working electrode
3: reference electrode
4: counter electrode
5: wiring
10: electrode
10a: untreated electrode
11: surface portion
11a: untreated surface portion
20: substrate
21: first surface of the substrate
30: reagent layer
31: outer peripheral edge portion of reagent layer
40: protective film
41: outer peripheral edge portion of protective film
50: insulating layer

52: opening
53: reference electrode opening
54: counter electrode opening
55: inner peripheral edge portion of opening in insulating layer
58: opening surrounding region
110: first region
111: first outer peripheral edge portion
112: center region
113: outer peripheral edge portion of center region
114: inner peripheral edge portion of surrounding region
115: surrounding region
116: connecting region
120: second region
121: second inner peripheral edge portion
122: second outer peripheral edge portion
130: third region
131: third inner peripheral edge portion
132: third outer peripheral edge portion
140: fourth region
141: fourth inner peripheral edge portion
142: fourth outer peripheral edge portion
150: fifth region
151: fifth inner peripheral edge portion
500: untreated region
510: first untreated region
520: second untreated region
530: third untreated region
540: fourth untreated region
C: cells
L: liquid sample
P: first liquid composition
Q: second liquid composition

## Claims

1. An electrode to be disposed on an insulating substrate, comprising:

   a surface portion that is positioned on the opposite side from the substrate when the electrode is disposed on the substrate;
   a first region that is formed on the surface portion, includes a first outer peripheral edge portion, and has a first surface free energy;
   a second region that is formed on the surface portion, surrounds the first region, includes a second inner peripheral edge portion in contact with the first outer peripheral edge portion of the first region, and a second outer peripheral edge portion positioned more to the outside than the second inner peripheral edge portion, and has a second surface free energy that is greater than the first surface free energy; and
   a third region that is formed on the surface portion, surrounds the second region, includes a third inner peripheral edge portion in contact with the second outer peripheral edge portion of the second region, and has a third surface free energy that is less than the second surface free energy.

2. The electrode according to claim 1,

   wherein the third region further includes a third outer peripheral edge portion located more to the outside than the third inner peripheral edge portion,
   said electrode further comprising a fourth region that is formed on the surface portion, surrounds the third region, includes a fourth inner peripheral edge portion in contact with the third outer peripheral edge portion of the third region, and a fourth outer peripheral edge portion located more to the outside than the fourth inner peripheral edge portion, and has a fourth surface free energy that is greater than the third surface free energy.

3. The electrode according to claim 2,
further comprising an insulating layer, at least part of which is disposed on the surface portion, and that includes an opening formed in the first region, the second region, the third region, and the fourth region, having an inner peripheral edge portion in contact with the fourth outer peripheral edge portion of the fourth region in plan view from a direction perpendicular to the surface portion, and passing through in the thickness direction.

4. The electrode according to claim 1 or 2,
further comprising an insulating layer, at least part of which is disposed on the surface portion, and that includes an opening formed in the first region, the second region, and the third region and passing through in the thickness direction.

5. An electrode to be disposed on an insulating substrate, comprising:

a surface portion that is positioned on the opposite side from the substrate when the electrode is disposed on the substrate;
a first region that is formed on the surface portion, includes a first outer peripheral edge portion, and has a first surface free energy;
a second region that is formed on the surface portion, surrounds the first region, includes a second inner peripheral edge portion in contact with the first outer peripheral edge portion of the first region, and a second outer peripheral edge portion positioned more to the outside than the second inner peripheral edge portion, and has a second surface free energy that is greater than the first surface free energy; and
an insulating layer, at least a portion of which is disposed on the surface portion, and that includes an opening formed in the first region and the second region, having an inner peripheral edge portion that defines the second outer peripheral edge portion of the second region, and passing through in the thickness direction.

6. A sensor, comprising:

the electrode according to any of claims 1 to 5; and
a reagent layer that is disposed in the first region and the second region of the electrode, and includes an outer peripheral edge portion positioned on the second outer peripheral edge portion of the second region, and a reagent participating in an oxidation-reduction reaction.

7. The sensor according to claim 6,
further comprising a protective film that covers the reagent layer.

8. The sensor according to claim 6 or 7,
further comprising an insulating substrate on which the electrode is disposed.

9. A method for manufacturing a sensor comprising:

the electrode according to any of claims 1 to 5; and
a reagent layer that is disposed in the first region and the second region of the electrode, and includes an outer peripheral edge portion positioned on the second outer peripheral edge portion of the second region, and a reagent participating in an oxidation-reduction reaction,
said method comprising:
coating the first region and the second region of the electrode with a first liquid composition containing the reagent in a first solvent, and then drying the first liquid composition to form the reagent layer.

10. The method according to claim 9,
wherein the first solvent includes one or more selected from among water and alcohol.

11. A method for manufacturing a sensor comprising:

the electrode according to any of claims 1 to 5;
a reagent layer that is disposed in the first region and the second region of the electrode, and includes an outer peripheral edge portion positioned on the second outer peripheral edge portion of the second region, and a reagent participating in an oxidation-reduction reaction; and
a protective film that covers the reagent layer,

said method comprising:

coating the first region and the second region of the electrode with a first liquid composition including the reagent in a first solvent, and then drying the first liquid composition to form the reagent layer, and coating the surface portion of the electrode with a second liquid composition containing a protective film component in a second solvent so as to cover the reagent layer, and then drying the second liquid composition to form the protective film.

12. The method according to claim 11,

wherein the electrode is the electrode according to claim 2 or 3, the protective film is disposed in the first region, the second region, the third region, and the fourth region of the electrode so as to cover the reagent layer, and includes an outer peripheral edge portion that is positioned on the fourth outer peripheral edge portion of the fourth region, and the formation of the protective film includes coating the first region, the second region, the third region, and the fourth region of the electrode with the second liquid composition so as to cover the reagent layer, and then drying the second liquid composition.

13. A method for manufacturing the electrode according to claim 3, comprising the steps of: preparing an untreated electrode including:

an untreated surface portion having an untreated region for forming the first region, the second region, the third region, and the fourth region in a part of the untreated surface portion and having the first surface free energy, and an insulating layer, at least part of which is disposed on the untreated surface portion, and that includes an opening formed in the untreated region and passing through in the thickness direction; and irradiating, out of the untreated region of the untreated surface portion in the untreated electrode, an annular fourth untreated region disposed on the outer periphery of the untreated region adjacent to the inner peripheral edge portion of the opening in the insulating layer and an annular second untreated region disposed further toward the inside than the fourth untreated region in a plan view from a direction perpendicular to the untreated surface portion, with a laser beam, thereby converting the fourth untreated region into the fourth region, and converting the second untreated region into the second region.

14. The method according to claim 13, wherein the irradiation of the fourth untreated region of the untreated region with the laser beam further includes irradiating an opening peripheral area of the insulating layer that surrounds the opening, which is adjacent to the outside of the fourth untreated region in a plan view from a direction perpendicular to the untreated surface portion, with the laser beam.

15. The method according to claim 14, wherein the irradiation of the opening peripheral area of the insulating layer with the laser beam further includes irradiating the opening peripheral area of the insulating layer with the laser beam to destroy the insulating layer in the opening peripheral area and convert the part of the untreated surface portion exposed by the destruction into the fourth region.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

LASER DESIGN

REAGENT LAYER

FIG. 28

FIG. 29A

FIG. 29B

FIG. 29C

BLANK TEST

Current [nA]

Time [Hr]

FIG. 29D

EP 4 567 415 A1

FIG. 29E

FIG. 30

EP 4 567 415 A1

FIG. 31

60

**FIG. 32**

**FIG. 33**

10A

FIG. 34

FIG. 35

FIG. 36

FIG. 37

FIG. 38

## INTERNATIONAL SEARCH REPORT

| | International application No. |
| --- | --- |
| | **PCT/JP2023/028101** |

| A. | CLASSIFICATION OF SUBJECT MATTER |
| --- | --- |

**G01N 27/327**(2006.01)i
FI: G01N27/327 353J

According to International Patent Classification (IPC) or to both national classification and IPC

| B. | FIELDS SEARCHED |
| --- | --- |

Minimum documentation searched (classification system followed by classification symbols)

G01N27/327; G01N27/416

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2007-278981 A (JAPAN ADVANCED INSTITUTE OF SCIENCE AND TECHNOLOGY HOKURIKU) 25 October 2007 (2007-10-25) paragraphs [0015], [0016]-[0024], [0027], fig. 2-4 | 1, 4, 6, 8-10 |
| Y | | 5-11 |
| Y | JP 2005-249530 A (YAMAHA CORPORATION) 15 September 2005 (2005-09-15) claims 1-2 | 5-11 |
| Y | WO 2019/176339 A1 (PHC HOLDINGS CORP.) 19 September 2019 (2019-09-19) claims, paragraphs [0008]-[0009] | 7, 11 |
| A | JP 2018-36091 A (ARKRAY, INC.) 08 March 2018 (2018-03-08) entire text, all drawings | 1-15 |
| A | JP 2015-200570 A (MURATA MANUFACTURING CO., LTD.) 12 November 2015 (2015-11-12) entire text, all drawings | 1-15 |
| A | JP 2005-10045 A (ARKRAY, INC.) 13 January 2005 (2005-01-13) entire text, all drawings | 1-15 |

☑ Further documents are listed in the continuation of Box C.  ☑ See patent family annex.

| | |
| --- | --- |
| *  Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| **21 September 2023** | **10 October 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2023/028101** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2011-13072 A (NIKKISO CO., LTD.) 20 January 2011 (2011-01-20)<br>entire text, all drawings | 1-15 |

Form PCT/ISA/210 (second sheet) (January 2015)

## INTERNATIONAL SEARCH REPORT

International application No.

**PCT/JP2023/028101**

**Box No. III** **Observations where unity of invention is lacking (Continuation of item 3 of first sheet)**

This International Searching Authority found multiple inventions in this international application, as follows:

(Invention 1) Claim 1 and claims 2-4, 6-15 depending from claim 1

Claim 1 and claims 2-4, 6-15 depending from claim 1 are classified as invention 1 as a result of having the special technical feature of comprising "a third region that includes a third inner peripheral edge portion which is formed on the surface section, surrounds the second region, and contacts the second outer peripheral edge portion of the second region, and that has a third free surface energy lower than the second free surface energy".

(Invention 2) Claim 5 and claims 6-11 depending from claim 5

Claim 5 shares, with claim 1 classified as invention 1, the feature of an "electrode disposed on an insulating substrate", comprising "a surface portion positioned on the side opposite to the substrate when the electrode is disposed on the substrate; a first region formed on the surface portion, including a first outer peripheral edge portion, and having a first free surface energy; and a second region formed on the surface portion and surrounding the first region, including a second inner peripheral edge portion which is in contact with the first outer peripheral edge portion of the first region and also including a second outer peripheral edge portion that is positioned more outside than the second inner peripheral edge portion, the second region having a second free surface energy that is greater than the first free surface energy".

However, this feature does not make a contribution over the prior art in the light of the content disclosed in document 1, and thus this feature cannot be said to be a special technical feature. Furthermore, there are no other identical or corresponding special technical features between these inventions.

Additionally, claim 5 does not depend from claim 1. Moreover, claim 5 is not substantially identical to or similarly closely related to any of the claims classified as invention 1.

Therefore, claim 5 and claims 6-11 depending from claim 5 cannot be classified as invention 1.

Claim 5 and claims 6-11 depending from claim 5 are classified as invention 2 as a result of having the special technical feature of comprising "an insulating layer at least a portion of which is disposed on the surface portion, the insulating layer having an inner peripheral edge portion formed on the first region and the second region, defining the second outer peripheral edge portion of the second region, and including an opening penetrating in the thickness direction".

Document 1: JP 2007-278981 A (JAPAN ADVANCED INSTITUTE OF SCIENCE AND TECHNOLOGY HOKURIKU) 25 October 2007 (2007-10-25)

paragraphs [0015], [0016]-[0024], [0027], fig. 2-4

(Family: none)

## INTERNATIONAL SEARCH REPORT

| International application No. |
|---|
| **PCT/JP2023/028101** |

**Box No. III     Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2023/028101**

| Patent document cited in search report | | | Publication date (day/month/year) | Patent family member(s) | Publication date (day/month/year) |
|---|---|---|---|---|---|
| JP | 2007-278981 | A | 25 October 2007 | (Family: none) | |
| JP | 2005-249530 | A | 15 September 2005 | (Family: none) | |
| WO | 2019/176339 | A1 | 19 September 2019 | US        2021/0000393        A1<br>claims, paragraphs [0008]-[0009]<br>EP              3766421      A1<br>CN          111867469          A | |
| JP | 2018-36091 | A | 08 March 2018 | US        2018/0057851        A1<br>whole document<br>EP              3290523      A1<br>CN          107796857          A | |
| JP | 2015-200570 | A | 12 November 2015 | (Family: none) | |
| JP | 2005-10045 | A | 13 January 2005 | (Family: none) | |
| JP | 2011-13072 | A | 20 January 2011 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010032501 A

- JP 2022124198 A